# EUROPEAN PATENT APPLICATION

(11) **EP 4 289 867 A1**
(43) Date of publication of application: **13.12.2023**
(21) Application number: 22748944.0
(22) Date of filing: 24.01.2022
(51) Int. Cl.: C07K 19/00, C12N 15/62, C12N 5/10, A61K 35/17, A61P 35/00

(54) **CHIMERIC ANTIGEN RECEPTOR WITH ENDOGENOUS PROTEIN MOLECULE REPLACING SINGLE DOMAIN ANTIBODY**

(30) Priority: 08.02.2021 CN 202110172889
(71) Applicant: Zhejiang University, Hangzhou, Zhejiang 310058 (CN)
(72) Inventor: YING, Songmin, Hangzhou, Zhejiang 310058 (CN); CHEN, Sisi, Hangzhou, Zhejiang 310058 (CN)
(74) Representative: Mewburn Ellis LLP
(86) International application number: PCT/CN2022/073560
(87) International publication number: WO 2022/166665

(57) **Abstract**

The present application provides an endogenous chimeric antigen receptor (ECAR). An antigen-binding domain in the endogenous chimeric antigen receptor is an endogenous protein molecule. The engineered immune cells in the present invention can kill a variety of cells specifically and selectively.

## Description

### Technical field

The present invention relates to the field of immunotherapy and, specifically, to a chimeric antigen receptor that replaces a single structural domain antibody with an endogenous protein molecule.

### Background

With the development of cellular immunotherapy and the success of clinical application, Chimeric Antigen Receptor T cell (CAR T) immunotherapy has become one of the most promising tumor immunotherapy pathways nowadays. Chimeric Antigen Receptor T cells (CAR T) are genetically modified T cells that can specifically recognize specific antigens in the body and kill the target cells where the specific antigens are located.

Chimeric antigen receptor (CAR) can be used to modify T lymphocytes to express chimeric antigen receptor through techniques such as viral infection, and such chimeric antigen receptor (CAR)-modified T cells are capable of specifically recognizing and binding to the target antigens in an MHC-unrestricted manner, and specifically killing the target cells. The chimeric antigen receptor (CAR) contains an extracellular antigen-binding domain, a transmembrane domain, and an intracellular signaling domain. While the extracellular antigen-binding domain is currently dominated by single-domain antibodies, specific single-domain antibodies are designed and prepared for specific target antigens, one of the reasons being that these target antigens are mainly tumor surface-specific markers, which lack corresponding ligands in vivo.

Furthermore, a clinical study of CAR T targeting mesothelin (2013) found that a single domain of a murine antibody on a chimeric antigen receptor triggered an anaphylactic reaction that resulted in the death of one of the subjects. This also illustrates the clinical rejection problems of chimeric antigen receptors with single structural domain antibodies as extracellular binding domains. In addition, the preparation process of chimeric antigen receptors with antibody single-chain variable fragments (scFv) as the extracellular antigen-binding structural domain is time-consuming, with high time cost, high economic cost, and difficulty in antibody screening.

Therefore, there is an urgent need in this field to develop a new chimeric antigen receptor that avoids rejection and is well tolerated.

### Summary of the invention

It is an object of the present invention to provide a new chimeric antigen receptor that avoids rejection and is well tolerated.

In a first aspect of the present invention, it provides an endogenous chimeric antigen receptor CAR (ECAR), comprising an antigen-binding domain, which is an endogenous protein molecule.

In another preferred example, the endogenous protein molecule is selected from the group consisting of: interleukin family, chemokine family, colony stimulating factor, growth factor, tumor necrosis factor superfamily, interferon family, tumor marker, senescent cells-associated factor, and a combination thereof.

In another preferred example, the interleukin family is selected from the group consisting of IL1α (interleukin 1α), IL1β (interleukin 1β), IL2 (interleukin 2), IL3 (interleukin 3), IL4 (interleukin 4), IL5 (interleukin 5), IL6 (interleukin 6), IL9 (interleukin 9), IL10 (interleukin 10), IL12 (interleukin 12), IL13 (interleukin 13), IL14 (interleukin 14), IL17A (interleukin 17A), IL17B (interleukin 17B), IL17C (interleukin 17C), IL17E (interleukin 17E), IL17F (interleukin 17F), IL33 (interleukin 33), and a combination thereof.

In another preferred example, the chemokine family is selected from the group consisting of CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL24, CCL25, CCL26, CCL27, CCL28, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17, XCL1, XCL2, CX3CL1, GRO/MGSA (melanoma cell growth-stimulating activity), PF-4 (Platelet Factor-4), Platelet Basic Protein, IP-10 (Inflammatory Protein 10), ENA-78, MIP-1α (macrophage inflammatory protein 1α), MIP-1β, MCP-1/MCAF (monocyte chemoattractant protein-1), MCP-2, MCP-3, and a combination thereof.

In another preferred example, the colony-stimulating factor is selected from the group consisting of G-CSF, M-CSF, GM-CSF, and a combination thereof.

In another preferred example, the growth factor is selected from the group consisting of: EGF (epidermal growth factor), VEGF (vascular endothelial cell growth factor), FGF (fibroblast growth factor), PDGF (platelet-derived endothelial cell growth factor), HGF (hepatocyte growth factor), IGF- I (insulin-like growth factor), IGF- II, LIF (leukaemia inhibitory factor), NGF (nerve growth factor), TGF-α (transforming growth factor), TGF-β1, TGF-β2, TGF-β3, TGFβ1β2, BMP (Bone Morphogenetic protein), and a combination thereof.

In another preferred example, the tumor necrosis factor is selected from the group consisting of: TNF-α, TNF-β, and a combination thereof.

In another preferred example, the interferon family is selected from the group consisting of: IFN-α (alpha interferon), IFN-β (beta interferon), IFN-y (gamma interferon), and a combination thereof.

In another preferred example, the tumor marker is selected from the group consisting of: CEA (serum carcinoembryonic antigen), AFP (alpha fetoprotein), PSA (prostate-specific antigen), and a combination thereof.

In another preferred example, the senescent cell-associated factor comprises Plau (urokinase-type plasminogen activator).

In another preferred example, the endogenous protein molecule comprises a wild type endogenous protein molecule and a mutant endogenous protein molecule.

In another preferred example, the mutant type comprises a mutant form in which the function of the encoded protein is unaltered (i.e. the function is the same or substantially the same as that of the wild-type encoded protein) and in which the function is enhanced after mutation.

In another preferred example, the endogenous protein molecule comprises a full-length protein or a protein fragment.

In another preferred example, the endogenous protein molecule protein is derived from a mammal, more preferably from a rodent (e.g. mouse, rat), a primate and a human.

In another preferred example, the endogenous protein molecule further comprises a derivative of the endogenous protein molecule.

In another preferred example, the derivative of an endogenous protein molecule comprises a modified endogenous protein molecule, a protein molecule with amino acid sequences homologous to natural endogenous protein molecules, a dimer or a multimer of an endogenous protein molecule, a fusion protein containing the amino acid sequence of an endogenous protein molecule.

In another preferred example, the modified endogenous protein molecule is a PEGylated endogenous protein molecule.

In another preferred embodiment, the "protein molecule with amino acid sequences homologous to natural endogenous protein molecules and with natural endogenous protein peptide activity " means a protein molecule having an amino acid sequence having ≥ 85% homology, preferably ≥ 90% homology, more preferably ≥ 95% homology, and most preferably ≥ 98% homology compared to an endogenous protein molecule; and having natural endogenous protein peptide activity.

In another preferred example, the polypeptide encoded by the gene of the mutant endogenous protein molecule is identical or substantially identical to the polypeptide encoded by the gene of the wild-type endogenous protein molecule.

In another preferred example, the gene of the mutant endogenous protein molecule comprises a polynucleotide having ≥80% homology (preferably ≥ 90%, more preferably ≥ 95%, most preferably ≥ 98% or 99%) compared to the gene of the wild-type endogenous protein molecule.

In another preferred example, the gene of the mutant endogenous protein molecule comprises a polynucleotide with 1-60 (preferably 1-30, more preferably 1-10) nucleotides truncated or added at the 5' end and/or the 3' end of the gene of the wild-type endogenous protein molecule.

In another preferred example, the amino acid sequence of the endogenous protein molecule is selected from the group consisting of:
(i) having an amino acid sequence as shown in any one of SEQ ID NO.: 1-5;
(ii) a polypeptide derived from (i) having the endogenous protein peptide activity, formed by substitution, deletion or addition of one or more amino acid residues to an amino acid sequence as shown in any one of SEQ ID NO.: 1-5; or
(iii) a polypeptide derived from (i) having the endogenous protein peptide activity, the amino acid sequence has ≥80% (preferably ≥90%, more preferably ^95% or ^98%) homology to any of the amino acid sequences as shown in SEQ ID NOs.: 1-5.

In another preferred example, the endogenous chimeric antigen receptor CAR (ECAR) has the structure shown in Formula I:

L-Z1-Z2-TM-C-CD3ζ (I)

wherein,
Each "-" is independently a linker peptide or peptide bond;
L is an optional signal peptide sequence;
Z1 is an antigen-binding domain, which is an endogenous protein molecule; and
Z2 is an absent or a hinge region;
TM is a transmembrane structural domain;
C is a co-stimulatory signaling molecule;
CD3ζ is a cytoplasmic signaling sequence derived from CD3ζ.

In another preferred example, the L is a signaling peptide of a protein selected from the group consisting of CD8, CD8α, CD28, GM-CSF, CD4, CD137, FcRy, FcRβ, CD3ζ, CD3γ, CD3δ, CD3ε, CD5, CD22, CD20, CD79a, CD79b, CD278(ICOS), FcERI, CD66d, DAP10, DAP12, and a combination thereof.

In another preferred example, the signal peptide of L is a signal peptide of human CD8 with the amino acid sequence MALPVTALLLPLALLLHAARP (SEQ ID NO.:16).

In another preferred example, the signal peptide of L is a signal peptide of murine origin CD8 with the amino acid sequence MASPLTRFLSLNLLLLGESIILGSGEA (SEQ ID NO.: 17).

In another preferred example, the Z2 is a hinge region of a protein selected from the group consisting of: a CD8, a CD8α, a CD28, a CD137, an Ig (immunoglobulin) hinge, and a combination thereof.

In another preferred example, the Z2 comprises a wild-type hinge region and a mutant hinge region.

In another preferred example, the mutant type comprises a fusion hinge region, or a combination thereof, following deletion, addition or substitution of one or several amino acids in the protein hinge region.

In another preferred example, the Z2 is a human CD28 hinge region with the amino acid sequence IEVMYPPPYLDNEKSNGTIIHVKGKHLCPSPLFPGPSKP (SEQ ID NO.:18).

In another preferred example, the Z2 is a human-derived CD8α hinge region, with the amino acid sequence TTTPAPRPPTPAPTIASQPLSLRPEACRPAAGGAVHTRGLDFACD (SEQ ID NO.:19).

In another preferred example, the Z2 is a murine-derived CD8α hinge region with the amino acid sequence STTTKPVLRTPSPVHPTGTSQPQRPEDCRPRGSVKGTGLDFACDIY (SEQ ID NO.:20).

In another preferred example, the TM is a transmembrane region of a protein selected from the group consisting of: CD28, CD3ε, CD45, CD4, CD5, CD8, CD9, CD16, CD22, CD33, CD37, CD64, CD80, CD86, CD134, CD137, CD154, CD8α, ICOS, CD19, CD45, and a combination thereof.

In another preferred example, the TM transmembrane region comprises a wild type protein transmembrane region and a mutant protein transmembrane region.

In another preferred example, the mutant type comprises a fusion protein transmembrane region, or a combination thereof, following deletion, addition or substitution of one or several amino acids in the protein transmembrane region.

In another preferred example, the TM is a transmembrane region selected from the human CD28 protein with the amino acid sequence FWVLVVVVGGVLACYSLLVTVAFIIFWV (SEQ ID NO.:21).

In another preferred example, the TM is a transmembrane region selected from a human CD8 protein, with the amino acid sequence: IYIWAPLAGTCGVLLLSLVIT (SEQ ID NO.:22).

In another preferred example, the TM is a transmembrane region selected from a murine-derived CD8 protein with the amino acid sequence: IWAPLAGICVALLLSLIITLI (SEQ ID NO.:23).

In another preferred example, the C is a co-stimulatory signalling molecule for a protein selected from the group consisting of: CD28, CD27, CD3ζ, CD3γ, CD3δ, CD3ε, CD5, CD22, CD79a, CD79b, CD66d, CD2, CD4, CD5, CD30, CD40, CD134, CD137, ICOS, CD154, 4-1BB, OX40, CD7, LIGHT, NKG2C, B7-H3, OX40, activated NK cell receptor, BTLA, Toll ligand receptor, CD2, CD7, CD27, CD30, CD40, CDS, ICAM-L LFA-1 (CD11a/CD18), B7-H3, CDS, ICAM-1, ICOS (CD278), GITR, BAFFR, HVEM (LIGHTR), KIRDS2, SLAMF7, NKp80 (KLRF1), NKp44, NKp30, NKp46, CD19, CD4, CD8α, CD8β, IL2Rβ, IL2Ry, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (tactile sense), CEACAM1, CRTAM, Ly9 (CD229), CD160 (BY55), PSGL1, CD100 (SEMA4D), CD69, SLAMF6 (NTBA, Ly108), SLAM (SLAMF1, CD150, IPO-3), BLAME (SLAMF8), SELPLG (CD162), LTBR, LAT, GADS, SLP-76, PAG/Cbp, CD19a, DAP10, DAP12, a ligand for CD83, MHC class I molecule, TNF receptor protein, immunoglobulin-like protein, cytokine receptor, integrin, signalling lymphocyte activation molecule, and a combination thereof.

In another preferred example, the C is a co-stimulatory signaling molecule of human CD28 origin with the amino acid sequence RSKRSRLLHSDYMNMTPRRPGPTRKHYQPYAPPRDFAAYRS (SEQ ID NO.:24).

In another preferred example, the C is a co-stimulatory signaling molecule of human 4-1BB origin with the amino acid sequence KRGRKKKLLYIFKQPFMRPVQTTQEEDGCSCRFPEEEEGGCEL (SEQ ID NO.:25).

In another preferred example, the C is a murine CD28-derived co-stimulatory signaling molecule with the amino acid sequence NSRRNRLLQSDYMNMTPRRPGLTRKPYQPYAPARDFAAYRP (SEQ ID NO.:26).

In another preferred example, the CD3ζ is the intracellular region of the CD3ζ protein molecule.

In another preferred example, the CD3ζ comprises the intracellular region of a wild-type CD3ζ protein molecule and the intracellular region of a mutant CD3ζ protein molecule.

In another preferred example, the mutant amino acid sequence comprises a fusion amino acid sequence, or a combination thereof, following the deletion, addition or substitution of one or several amino acids in the amino acid sequence of the intracellular region of the CD3ζ protein molecule.

In another preferred example, the CD3ζ is the intracellular region of a human-derived CD3ζ protein molecule with the amino acid sequence:

RVKFSRSADAPAYKQGQNQLYNELNLGRREEYDVLDKRRGRDPEMGGKPR
RKNPQEGLYNELQKDKMAEAYSEIGMKGERRRGKGHDGLYQGLSTATKDT
YDALHMQALPPR (SEQ ID NO.: 27).

In another preferred example, the CD3ζ is the intracellular region of the murine-derived CD3ζ protein molecule with the amino acid sequence:

RAKFSRSAETAANLQDPNQLYNELNLGRREEYDVLEKRARDPEMGGKQQR
RRNPQEGVYNALQKDKMAEAYSEIGTKGERRRGKGHDGLYQGLSTATKDT
YDALHMQTLAPR (SEQ ID NO.: 28).

In another preferred example, the amino acid sequence of the endogenous chimeric antigen receptor CAR (ECAR) is as shown in any one of SEQ ID NO.:6-10.

In a second aspect of the present invention, it provides a nucleic acid molecule encoding an endogenous chimeric antigen receptor CAR (ECAR) as described in the first aspect of the present invention.

In another preferred example, the nucleic acid molecule is selected from the group consisting of:
(a) a polynucleotide encoding a polypeptide as shown in any of SEQ ID NO: 1-5;
(b) a polynucleotide with a sequence as shown in any of SEQ ID NO: 11-15;
(c) a polynucleotide having a nucleotide sequence having ^75% (preferably ≥ 80%, more preferably, 90%, more preferably, 95%, more preferably, 98%, more preferably, 99%) homology to the sequence as shown in (b);
(d) a polynucleotide with the 5' end and/or 3' end of the polynucleotide as shown in (b) truncated or with 1-60 (preferably, 1-30, more preferably 1-10) nucleotides added;
(e) a polynucleotide complementary to the polynucleotide described in any one of (a)-(d).

In another preferred example, the nucleotide sequence of the nucleic acid molecule is shown in any one of SEQ ID NO: 11-15.

In another preferred example, the nucleic acid molecule is a polynucleotide.

In a third aspect of the present invention, it provides a vector, comprising a nucleic acid molecule as described in the second aspect of the present invention.

In another preferred example, the vector is selected from the group consisting of: a plasmid, a lentiviral vector, an adenoviral vector, a retroviral vector, and a combination thereof.

In another preferred example, the vector is a lentiviral vector.

In another preferred example, the vector is a retroviral vector.

In a fourth aspect of the present invention, it provides a host cell, comprising a vector as described in the third aspect of the present invention or a chromosome incorporating an exogenous nucleic acid molecule as described in the second aspect of the present invention or expressing an ECAR as described in the first aspect of the present invention.

In another preferred example, the cell is an isolated cell, and/or the cell is a genetically engineered cell.

In another preferred example, the cell is a mammalian cell.

In another preferred example, the cell is a macrophage, T cell or NK cell.

In another preferred example, the host cell is an engineered immune cell.

In another preferred example, the engineered immune cell comprises a macrophage, T-cell or NK-cell, preferably (i) an endogenous chimeric antigen receptor T-cell (ECAR-T-cell); (ii) an endogenous chimeric antigen receptor NK-cell (ECAR-NK-cell); (iii) an exogenous T-cell receptor (TCR) T-cell (TCR-T cell) or (iv) a endogenous chimeric antigen receptor macrophage (ECAR-macrophage).

In another preferred example, the immune cell is autologous.

In another preferred example, the immune cell is non-autologous.

In another preferred example, the mmune cell targets a target cell expressing a receptor matching an endogenous protein molecule.

In a fifth aspect of the present invention, it provides a method of preparing an engineered immune cell, the engineered immune cell expressing an ECAR as described in the first aspect of the present invention, comprising the step of transducing a nucleic acid molecule as described in the second aspect of the present invention or a vector as described in the third aspect of the present invention into a macrophage, a T-cell, or an NK-cell, thereby obtaining the engineered immune cell.

In another preferred example, the transducing comprises simultaneous, successive, or sequential transduction.

In another preferred example, the cell is an ECAR-macrophage, ECAR-T cell or ECAR-NK cell.

In another preferred example, the method further comprises the step of testing the obtained engineered immune cell for function and effectiveness.

In a sixth aspect of the present invention, it provides a pharmaceutical composition, comprising an ECAR as described in the first aspect of the present invention, a nucleic acid molecule as described in the second aspect of the present invention, a vector as described in the third aspect of the present invention, or a host cell as described in the fourth aspect of the present invention, as well as a pharmaceutically acceptable carrier, diluent or excipient.

In another preferred example, the pharmaceutical composition is a liquid formulation.

In another preferred example, the dosage form of the pharmaceutical composition is an injection.

In another preferred example, the concentration of the cell in the pharmaceutical composition is 1 × 10⁵-1 × 10⁸ cells/mL, preferably, 1 × 10⁶-1 × 10⁷ cells/mL, more preferably, 1 × 10⁶-5 × 10⁶ cells/mL.

In another preferred example, the pharmaceutical composition further comprises other drugs (e.g., antibody drugs, other CAR-T drugs, or chemotherapeutic drugs) that kill cells.

In another preferred example, the pharmaceutical composition further contains drugs that modulate the expression of ECAR as described in the first aspect of the present invention, drugs that modulate the killing function and cellular activity of host cells as described in the fourth aspect of the present invention, drugs that modulate the immune response in the host, drugs that modulate side effects (e.g., inhibitors of the T-cell apoptosis signaling pathway, neutralizing antibodies against cytokine storm).

In a seventh aspect of the present invention, it provides a use of an ECAR as described in the first aspect of the present invention, a nucleic acid molecule as described in the second aspect of the present invention, a vector as described in the third aspect of the present invention, a host cell as described in the fourth aspect of the present invention, or a pharmaceutical composition as described in the sixth aspect of the present invention for the preparation of a drug or formulation for (a) selective killing of a cell; and/or (b) treating a disease .

In another preferred example, the cell contains a receptor that matches an endogenous protein molecule.

In another preferred example, the cell is selected from the group consisting of: an inflammatory cell, senescent cell, tumor cell, autoimmune cell, and a combination thereof.

In another preferred example, the disease is selected from the group consisting of: neoplastic disease, allergic disease, autoimmune disease, senescent cell-associated disease, and a combination thereof.

In another preferred example, the neoplastic disease is selected from the group consisting of: malignant/benign tumors of epithelial cell origin, malignant/benign tumors of mesenchyme cell origin, malignant/benign tumors of hematopoietic stem cell origin, malignant/benign tumors of neuroepithelial cell origin.

In another preferred example, the allergic disease is selected from the group consisting of: skin allergic disease, respiratory allergic disease, allergic diseases of digestive tract, anaphylaxis, and a combination thereof.

In another preferred example, the autoimmune disease is selected from the group consisting of: rheumatoid arthritis, systemic lupus erythematosus, psoriasis, type I diabetes mellitus, inflammatory bowel disease, celiac disease, multiple sclerosis, hypoplastic anemia, diffuse toxic goiter, and a combination thereof.

In another preferred example, the aging-associated disease is selected from the group consisting of: aging-associated hepatic fibrosis, aging-associated pulmonary fibrosis, aging-associated atherosclerosis, aging-associated diabetes mellitus, aging-associated osteoarthritis, aging-associated sarcopenia, aging-associated obesity, and aging-associated glaucoma.

In an eighth aspect of the present invention, it provides a kit for selective cell killing, comprising a container, and an ECAR as described in the first aspect of the present invention, a nucleic acid molecule as described in the second aspect of the present invention, a vector as described in the third aspect of the present invention, a host cell as described in the fourth aspect of the present invention, or a pharmaceutical composition as described in the sixth aspect of the present invention in the container.

In another preferred example, the kit further contains a label or instructions for use.

In a ninth aspect of the present invention, it provides a method of selectively killing cells, comprising:
administering to a subject in need of treatment a safe and effective amount of an ECAR as described in the first aspect of the present invention, a host cell as described in the fourth aspect of the present invention, or a pharmaceutical composition as described in the sixth aspect of the present invention.

In another preferred example, the subject comprises a human or non-human mammal.

In another preferred example, the non-human mammal comprises a rodent (e.g. mouse, rat, rabbit), a primate (e.g. monkey), a suidae animal.

In another preferred example, the method is non-therapeutic and non-diagnostic.

In a tenth aspect of the present invention, it provides a method of treating a disease, comprising administering to a subject in need of treatment a safe and effective amount of an ECAR as described in the first aspect of the present invention, a host cell as described in the fourth aspect of the present invention or a pharmaceutical composition as described in the sixth aspect of the present invention.

In another preferred example, the method further comprises administering to the subject in need of treatment other drugs for the treatment of a neoplastic disease, allergic disease, autoimmune disease, senescent cell-associated disease.

In another preferred example, the other drugs include other drugs that kill cells (e.g., antibody drugs, other CAR-T drugs, or chemotherapeutic drugs), drugs that modulate the expression of endogenous chimeric antigen receptors, drugs that modulate the killing function and cellular activity of host cells, drugs that modulate the immune response in the host, drugs that modulate side effects, CAR-T drugs.

In another preferred example, the disease is selected from the group consisting of: neoplastic disease, allergic disease, autoimmune disease, senescent cell-associated disease, and a combination thereof.

In another preferred example, the neoplastic disease is selected from the group consisting of: malignant/benign tumors of epithelial cell origin, malignant/benign tumors of mesenchyme cell origin, malignant/benign tumors of hematopoietic stem cell origin, malignant/benign tumors of neuroepithelial cell origin.

In another preferred example, the allergic disease is selected from the group consisting of: skin allergic disease, respiratory allergic disease, allergic diseases of digestive tract, anaphylaxis, and a combination thereof.

In another preferred example, the autoimmune disease is selected from the group consisting of: rheumatoid arthritis, systemic lupus erythematosus, psoriasis, type I diabetes mellitus, inflammatory bowel disease, celiac disease, multiple sclerosis, hypoplastic anemia, diffuse toxic goiter, and a combination thereof.

In another preferred example, the aging-associated disease is selected from the group consisting of: aging-associated hepatic fibrosis, aging-associated pulmonary fibrosis, aging-associated atherosclerosis, aging-associated diabetes mellitus, aging-associated osteoarthritis, aging-associated sarcopenia, aging-associated obesity, and aging-associated glaucoma.

It should be understood that within the scope of the present invention, the above-mentioned technical features of the present invention and the technical features described in detail below (such as embodiments) can be combined with each other to form a new or preferred technical solution. Due to space constraints, I will not repeat them here.

### Description of Drawings

Figure 1 shows the hIL5-ECAR plasmid map.
Figure 2 shows the activation assay of hIL5-ECAR Jurkat cell line after co-culture with hIL5Ra-expressing target cells.
Figure 3 shows the CAR expression efficiency of hIL5-ECAR T-cell lentivirus after centrifugal infection of human T cells for 72 hours.
Figure 4 shows the specific killing effect of hIL5-ECAR T after co-culture with hILSRa-expressing target cells.
Figure 5 shows the IFN-y secretion of hIL5-ECAR T after co-culture with hIL5Ra-expressing target cells.
Figure 6 shows the mILS-ECAR plasmid map.
Figure 7 shows the CAR expression efficiency of mILS-ECAR retrovirus after centrifugal infection of mouse T cells for 48 hours.
Figure 8 shows the killing effect of mIL5-ECAR T on eosinophils after back-infusion into mouse asthma model.
Figure 9 shows the mitigative effect of mIL5-ECAR T on inflammatory factors after back infusion in a mouse model of asthma.
Figure 10 shows the mitigative effect on inflammation after mILS-ECAR T infusion back into a mouse model of asthma.
Figure 11 shows the hPlau-ECAR plasmid map.
Figure 12 shows the specific killing effect of hPlau-ECAR T after co-culture with hPlauR-expressing target cells.
Figure 13 shows a hCCL 11-ECAR plasmid map.
Figure 14 shows the specific killing effect of hCCL 11-ECAR T after co-culture with hCCR3-expressing target cells.
Figure 15 shows a hCCL24-ECAR plasmid map.
Figure 16 shows the specific killing effect of hCCL24-ECAR T after co-culture with hCCR3-expressing target cells.

### DETAILED DESCRIPTION

The present inventor, after extensive and in-depth research and after a large number of screenings, has for the first time accidentally discovered the design of endogenous chimeric antigen receptor (Endogenous CAR, ECAR) by using endogenous protein molecules instead of antibody single domain fragments as the extracellular antigen-binding domains of CAR, and after its expression in T cells, NK cells, macrophages, etc. and loading on membrane, can enable the above cells to specifically and selectively kill target cells and has a significant killing effect, and can also treat diseases, such as neoplastic diseases, allergic diseases, autoimmune diseases, and senescent cell-associated diseases. On this basis, the present inventor has accomplished the present invention.

The present invention provides a detailed description of the engineered immune cells of the present invention, representatively using ECAR-T cells as an example. The engineered immune cells of the present invention are not limited to the ECAR-T cells described in the context, and the engineered immune cells of the present invention have the same or similar technical features and beneficial effects as the ECAR-T cells described in the context. Specifically, when the immune cell expresses the endogenous chimeric antigen receptor ECAR, the macrophage, NK cell is equivalent to the T cell (or the T cell may replace the NK cell, macrophage); and when the immune cell is a T cell, the TCR is equivalent to the ECAR (or the ECAR may be replaced by the TCR).

### Endogenous protein molecules

In the present invention, an endogenous protein molecule refers to a peptide chain or protein molecule made up of amino acids by "dehydration synthesis" that are capable of being transcribed and translated in the human body using the human genome as a translation template. These protein molecules are multimers of 20 different amino acids linked together and have a three-dimensional spatial structure. In the physiological state, most protein molecules have the ability to bind specifically to other protein molecules, which plays an important role in protein interactions and in the pathological state, specific protein receptors are expressed on the surface of cells that promote pathological processes. Based on this, chimeric antigen receptors (ECAR) linking endogenous protein molecules have been designed and expressed in killer cells, such as T-cells, in order to induce ECAR killer cells to exert specific killing and removal of these harmful cells that promote the onset of pathological processes.

In a preferred embodiment of the present invention, the endogenous protein molecule of the present invention is preferably of the type of interleukin family, chemokine family, colony-stimulating factor, growth factor, tumor necrosis factor superfamily, interferon family, tumor marker, senescent cell-associated factor, and the like.

The present invention relates to an endogenous protein molecule and variants thereof.

In a preferred embodiment of the present invention, the amino acid sequence of the endogenous protein molecule of the present invention is as shown in any one of SEQ ID NO.:1-5.

IPTEIPTSALVKETLALLSTHRTLLIANETLRIPVPVHKNHQLCTEEIFQGI
GTLESQTVQGGTVERLFKNLSLIKKYIDGQKKKCGEERRRVNQFLDYLQEFL
GVMNTEWIIES (SEQ ID NO.: 1, from human-derived interleukin 5 factor IL5);

MEIPMSTVVKETLTQLSAHRALLTSNETMRLPVPTHKNHQLCIGEIFQG
LDILKNQTVRGGTVEMLFQNLSLIKKYIDRQKEKCGEERRRTRQFLDYLQEF
LGVMSTEWAMEG (SEQ ID NO.: 2, from mouse-derived interleukin 5 factor IL5)

SNELHQVPSNCDCLNGGTCVSNKYFSNIHWCNCPKKFGGQHCEIDKSK
TCYEGNGHFYRGKASTDTMGRPCLPWNSATVLQQTYHAHRSDALQLGLGK
HNYCRNPDNRRRPWCYVQVGLKLLVQECMVHDCADGKKPSSPPEELKFQC
GQKTLRPRFKIIGGEFTTIENQPWFAAIYRRHRGGSVTYVCGGSLISPCWVISA
THCFIDYPKKEDYIVYLGRSRLNSNTQGEMKFEVENLILHKDYSADTLAHHN
DIALLKIRSKEGRCAQPSRTIQTICLPSMYNDPQFGTSCEITGFGKENSTDYLY
PEQLKMTVVKLISHRECQQPHYYGSEVTTKMLCAADPQWKTDSCQGDSGGP
LVCSLQGRMTLTGIVSWGRGCALKDKPGVYTRVSHFLPWIRSHTKEENGLA
L (SEQ ID NO.: 3, from human urokinase-type plasminogen activator Plau)

GPASVPTTCCFNLANRKIPLQRLESYRRITSGKCPQKAVIFKTKLAKDIC
ADPKKKWVQDSMKYLDQKSPTPKP (SEQ ID NO.: 4, from the human CCL11
factor);

VVIPSPCCMFFVSKRIPENRVVSYQLSSRSTCLKAGVIFTTKKGQQFCGD
PKQEWVQRYMKNLDAKQKKASPRARAVAVKGPVQRYPGNQTTC (SEQ ID
NO.:5, from the human CCL24 factor)

The present invention further comprises polypeptides or proteins having the same or similar function that are 50% or more (preferably more than 60%, more than 70%, more than 80%, more preferably more than 90%, more preferably more than 95%, most preferably more than 98%, e.g. 99%) homologous to any of the sequences shown in SEQ ID NOs.: 1-5 of the present invention.

The protein of the present invention may be a recombinant protein, a natural protein, a synthetic protein. The proteins of the present invention may be products of natural purification, or chemical synthesis, or produced from prokaryotic or eukaryotic hosts (e.g., bacteria, yeast, higher plants, insect and mammalian cells) using recombinant techniques. Depending on the host used for the recombinant production scheme, the proteins of the invention may be glycosylated or may be non-glycosylated. The proteins of the present invention may also include or not include a starting methionine residue.

The present invention also includes endogenous protein molecule fragments and analogs having endogenous protein molecule activity. As used herein, the terms "fragments" and "analogs" refer to proteins that retain essentially the same biological function or activity as the natural endogenous protein molecule of the invention.

The mutant protein fragments, derivatives or analogs of the present invention may be (i) mutant proteins having one or more conserved or non-conserved amino acid residues (preferably conserved amino acid residues) substituted, and such substituted amino acid residues may or may not be encoded by the genetic code, or (ii) mutant proteins having substituents in one or more amino acid residues, or (iii) mutant protein formed by fusion of a mature mutant protein with another compound (e.g., a compound that extends the half-life of the mutant protein, such as polyethylene glycol), or (iv) mutant protein formed by fusing additional amino acid sequences to this mutant protein sequence (such as a leading sequence or a secreted sequence or a sequence used to purify this mutant protein or a proteinogen sequence, or a fusion protein formed with an antigen IgG fragment). According to the teachings herein, these fragments, derivatives, and analogs fall within the scope of what is known to those skilled in the art. In the present invention, the amino acids of the conservative substitutions are preferably generated by making amino acid substitutions according to Table I.

**Table I**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gin (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also comprises polypeptides or proteins having the same or similar function that are 50% or more (preferably more than 60%, more than 70%, more than 80%, more preferably more than 90%, more preferably more than 95%, most preferably more than 98%, e.g. 99%) homologous to the natural endogenous protein molecules of the present invention. In the protein variant, a derivative sequence obtained by several (typically 1-60, preferably 1-30, more preferably 1-20, most preferably 1-10) of substitutions, deletions or additions of at least one amino acid, as well as the addition of one or several (usually up to 20, preferably up to 10, more preferably up to 5) amino acids at the C-terminus and/or the N-terminus. For example, substitutions in the protein with amino acids having close or similar properties usually do not alter the function of the protein, and the addition of one or several amino acids to the C-terminus and/or the N-terminus usually does not alter the function of the protein. The present invention includes analogs of natural endogenous protein molecules that differ from the natural endogenous protein molecule either in amino acid sequence, in the form of modifications that do not affect the sequence, or both. These protein analogs include natural or induced genetic variants. Induced variants can be obtained by various techniques, such as random mutagenesis by radiation or exposure to mutagens, but also by targeted mutagenesis or other techniques known to molecular biology. Analogs also include analogs having residues different from natural L-amino acids (e.g., D-amino acids), and analogs having amino acids that are not naturally occurring or synthesized (e.g., β, γ-amino acids). It should be understood that the proteins of the present invention are not limited to the representative proteins exemplified above.

Forms of modification (which typically do not alter primary structure) include: chemically derived forms of the protein in vivo or in vitro such as acetylation or carboxylation. Modifications also include glycosylation, such as those performed in protein synthesis and processing. Such modifications may be accomplished by exposing the protein to an enzyme that performs glycosylation (e.g., a mammalian glycosylase or deglycosylase). Modified forms also include sequences having phosphorylated amino acid residues (e.g., phosphotyrosine, phosphoserine, phosphothreonine). In addition, modifications can be made to mutant proteins of the present invention. Forms of modification (which typically do not alter primary structure) include: chemically derived forms of the mutant protein in vivo or in vitro such as acetylation or carboxylation. Modifications also include glycosylation, such as those produced by glycosylation modifications in the synthesis and processing of mutant proteins or in further processing steps. Such modifications can be accomplished by exposing the mutant protein to an enzyme that performs glycosylation (e.g., a mammalian glycosylase or deglycosylase). Modified forms also include sequences having phosphorylated amino acid residues (e.g., phosphotyrosine, phosphoserine, phosphothreonine). Also included are mutant proteins that are modified so as to improve their resistance to protein hydrolysis or optimize solubility properties.

The present invention also provides polynucleotide sequences encoding endogenous protein molecules. The polynucleotides of the present invention may be in the form of DNA or in the form of RNA. the form of DNA includes: DNA, genomic DNA, or synthetic DNA, and the DNA may be single-stranded or double-stranded. Polynucleotides encoding a mature polypeptide include: coding sequences encoding only the mature polypeptide; coding sequences of the mature polypeptide and various additional coding sequences; coding sequences of the mature polypeptide (and optionally additional coding sequences) and non-coding sequences. The term "polynucleotide encoding a polypeptide" may be a polynucleotide comprising a polypeptide encoding such a polypeptide or a polynucleotide comprising additional coding and/or non-coding sequences. The present invention also relates to variants of the above-described polynucleotide which encode fragments, analogs and derivatives of polypeptides having the same amino acid sequence as the present invention. The variants of this polynucleotide may be naturally occurring allelic variants or non-naturally occurring variants. These nucleotide variants include substitution variants, deletion variants, and insertion variants. As is known in the art, an allelic variant is a polynucleotide substitution form, which may be a substitution, deletion, or insertion of one or more nucleotides without substantially altering the function of the polypeptide that it encodes.

Based on the nucleotide sequences described herein, a person skilled in the art can readily produce the encoded nucleic acids of the present invention by a variety of known methods. These methods include, for example, but are not limited to: PCR, DNA synthesis, etc., and specific methods can be found in J. Sambrook, Experimental Guide to Molecular Cloning. As an embodiment of the present invention, the coding nucleic acid sequences of the present invention may be constructed by means of segmented synthesized nucleotide sequences followed by overlapping extension PCR.

The present invention also relates to polynucleotides that hybridize to the sequences described above and have at least 50%, preferably at least 70%, more preferably at least 80% identity between the two sequences. The present invention relates in particular to polynucleotides which are hybridizable to the polynucleotides described herein under stringent conditions (or severe conditions). In the present invention, "stringent conditions" means (1) hybridization and elution at lower ionic strengths and higher temperatures, e.g., 0.2 × SSC, 0.1% SDS, 60° C; or (2) hybridization with a denaturant, e.g., 50% (v/v) formamide, 0.1% calf serum/0.1% Ficoll, 42° C., etc.; or (3) hybridization occurs only when the identity between the two sequences is at least 90%, preferably 95% or more.

The proteins and polynucleotides of the present invention are preferably provided in isolated form and, preferably, purified to homogeneity.

The full-length sequences of the polynucleotides of the present invention can typically be obtained by PCR amplification methods, recombinant methods or synthetic methods. For the PCR amplification method, primers can be designed according to the relevant nucleotide sequences disclosed herein, in particular the open reading frame sequences, and the sequences in question are amplified using commercially available cDNA libraries or cDNA libraries prepared according to conventional methods known to those skilled in the art as templates. When the sequence is long, it is often necessary to perform two or more PCR amplifications before splicing the fragments from each amplification in the correct order.

Once the sequence in question has been obtained, it can be obtained in large quantities by recombination. This is usually done by cloning it into a vector, transferring into cells, and then isolating the sequence in question from the proliferating host cells by conventional methods.

In addition, synthetic methods can be used to synthesize the sequence in question, especially if the fragments are short. Typically, very long fragments can be obtained by first synthesizing a number of small fragments and then ligating them.

Currently, DNA sequences encoding proteins (or fragments thereof, or derivatives thereof) of the present invention are already available exclusively by chemical synthesis. This DNA sequence can then be introduced into various existing DNA molecules (or e.g. vectors) and cells known in the art. In addition, mutations may be introduced into the protein sequences of the present invention by chemical synthesis.

The application of PCR techniques to amplify DNA/RNA is preferred for obtaining the polynucleotides of the present invention. In particular, when it is difficult to obtain full-length cDNA from a library, the RACE method Rapid amplification of cDNA Ends, RACE) may be preferably used, and primers for PCR may be appropriately selected on the basis of the sequence information of the present invention disclosed herein and may be synthesized by conventional methods. Amplified DNA/RNA fragments may be isolated and purified by conventional methods such as by gel electrophoresis.

### Antigen-binding domains

In the present invention, the antigen-binding domain of a chimeric antigen receptor CAR specifically binds to receptors (e.g., receptors for IL-5, IL-17) on the cell surface that can be matched to endogenous protein molecules.

### Hinge and transmembrane regions

For hinge regions and transmembrane regions (transmembrane domains), the CAR may be designed to include transmembrane domains fused to the extracellular domains of the CAR. In one embodiment, a transmembrane domain that is naturally associated with one of the domains in the CAR is used. In some examples, the transmembrane domain may be selected, or modified by amino acid substitutions, to avoid binding such a domain to the transmembrane domain of the same or a different surface membrane protein, thereby minimizing interaction with other members of the receptor complex.

The transmembrane domain may originate from a natural source or a synthetic source. In a natural source, the domain may originate from any membrane binding protein or transmembrane protein. Preferably, the hinge region in the CAR of the present invention is the hinge region of CD8α or the CD28 length hinge region, and the transmembrane region of the present invention is the transmembrane region of CD8α or the transmembrane region of CD28.

### Intracellular domains

The intracellular domain of the CAR of the present invention, or an additional intracellular signaling domain is responsible for the activation of at least one normal effector function of the immune cell in which the CAR has been placed. The term "effector function" refers to a cell-specific function. For example, an effector function of a T cell may be a cytolysis or auxiliary activity that includes cytokine secretion. Thus, the term "intracellular signaling domain" refers to the portion of the protein that transduces the effector function signal and directs the cell to perform the proprietary function. Although the entire intracellular signaling domain can often be used, in many examples it is not necessary to use the entire chain. In terms of using truncated portions of intracellular signaling sdomains, such a truncated portion may be used in place of the intact chain as long as it transduces effector function signals. The term intracellular signaling domain thus refers to any truncated portion of comprising an intracellular signaling domain sufficient to transduce a functional signal from an effector.

Preferred examples of intracellular signaling domains for use in the CARs of the present invention include cytoplasmic sequences of T cell receptors (TCRs) and co-receptors that act in concert to initiate signal transduction upon antigen receptor binding, as well as any derivatives or variations of these sequences and any synthetic sequences that have the same functional capacity.

In preferred embodiments, the cytoplasmic domain of the CAR may be designed to include a CD3ζ signaling domain by itself, or may be in association with any other desired cytoplasmic domain (one or more) useful in the context of the CAR of the present invention. For example, the cytoplasmic domain of the CAR may include a CD3ζ chain portion and a co-stimulatory signaling region. The co-stimulatory signaling region refers to a portion of CAR that includes the intracellular domain of co-stimulatory molecules. Co-stimulatory molecules are cell surface molecules that are required for an effective response of a lymphocyte to an antigen, rather than antigen receptors or their ligands. Preferably, this includes CD28, 4-1BB, and the like.

The cytoplasmic signaling sequences within the cytoplasmic signaling portion of the CAR of the present invention may be randomly or in a defined order interconnected. Optionally, short oligopeptide or polypeptide linkers, preferably between 2 and 10 amino acids in length, may form the linkage. Glycine-serine duplexes provide particularly suitable linkers.

In one embodiment, the cytoplasmic domains in the CAR of the present invention are designed to include a signaling domain of CD28 (a co-stimulatory molecule) as well as a signaling domain of CD3ζ.

### Chimeric antigen receptors (CARs)

Chimeric antigen receptors (CARs) consist of an extracellular antigen recognition region, usually scFv (single-chain variable fragment), a transmembrane region, and an intracellular co-stimulatory signaling region.The design of CARs has gone through the following process: the first generation of CARs had only one intracellular signaling component, CD3ζ or FcyRI molecule, and because there was only one intracellular activation domain, it could only cause transient T cell proliferation and less cytokine secretion, but could not provide prolonged T cell proliferation signaling and sustained anti-tumor effect in vivo, so it did not achieve good clinical efficacy. The second generation CARs introduced a co-stimulatory molecule, such as CD28, 4-1BB, OX40, ICOS, on the basis of the original structure, which greatly improved the function compared with the first generation CARs, and further strengthened the persistence of CAR-T cells and the killing ability of tumor cells. Tandeming some new immune co-stimulatory molecules such as CD27 and CD134 on the basis of second-generation CARs, they develop into third- and fourth-generation CARs.

The extracellular segment of CARs recognizes a specific antigen and subsequently transduces this signal through intracellular domains, causing cell activation and proliferation, cytolytic toxicity, and secretion of cytokines, which in turn removes the target cells. Patient autologous cells (or heterologous donors) are first isolated, activated and genetically modified to produce CAR immune cells, which are subsequently injected into the same patient. This approach has a very low probability of developing graft-versus-host disease, and the antigen is recognized by the immune cells in a non-MHC-restricted manner.

CAR-immune cell therapy has achieved very high clinical response rates in the treatment of hematologic malignancies, such high response rates being unattainable by any previous therapeutic means, and has triggered a boom in clinical research around the world.

Specifically, the endogenous chimeric antigen receptor (ECAR) of the present invention includes an extracellular domain, a transmembrane domain, and an intracellular domain. The extracellular domain includes a target-specific binding element (also known as an antigen-binding domain). The intracellular domain includes a co-stimulatory signaling region and/or a ζ-chain portion. Co-stimulatory signaling region refers to a portion of the intracellular domain that includes a co-stimulatory molecule. The co-stimulatory molecules are cell surface molecules that are required for an effective response of the lymphocyte to the antigen, and not antigen receptors or their ligands.

A junction may be incorporated between the extracellular domain and the transmembrane domain of the CAR, or between the cytoplasmic domain and the transmembrane domain of the CAR. As used herein, the term "junction" generally refers to any oligopeptide or polypeptide that serves to connect the transmembrane domain to the extracellular or plasma domain of a polypeptide chain. The junction may comprise from 0 to 300 amino acids, preferably from 2 to 100 amino acids and most preferably from 3 to 50 amino acids.

The ECAR of the present invention when expressed in T cells is capable of antigen recognition based on antigen binding specificity. When the ECAR binds antigens specific to the surface of the target cell, it is able to activate the ECAR T cell and cause the ECAR T cell to kill the target cell. The antigen-binding domain is preferably fused to an intracellular domain derived from one or more of a co-stimulatory molecule and/or a ζ-chain. Preferably, the antigen-binding domain is fused to an intracellular domain of a combination of a CD28 signaling domain and/or a CD3ζ signaling domain.

In a preferred embodiment, the antigen-binding portion of the ECAR of the present invention targets a receptor corresponding (matching) to the endogenous protein molecule. In a preferred embodiment, the antigen-binding portion of the ECAR of the present invention is an endogenous protein molecule targeting receptors matching endogenous protein molecules.

In a preferred embodiment, the endogenous protein molecule comprises a variant form, said variant having ≥80%, ^85%, ≥90%, ≥95%, ≥98%, or ≥ 99% homology to the protein sequence of its wild type endogenous protein molecule.

In the present invention, the endogenous protein molecule of the present invention further comprises a conserved variant thereof, meaning that up to 10, preferably up to 8, better up to 5, optimally up to 3 amino acids have been replaced by amino acids of a close or similar nature to form a polypeptide compared to the amino acid sequence of the endogenous protein molecule of the present invention.

In the present invention, the number of said added, deleted, modified and/or substituted amino acids is preferably not more than 40% of the total number of amino acids in the initial amino acid sequence, more preferably not more than 35%, more preferably 1-33%, more preferably 5-30%, more preferably 10-25%, more preferably 15-20%.

In the present invention, the number of said added, deleted, modified and/or substituted amino acids is typically 1, 2, 3, 4 or 5, preferably 1-3, better 1-2, optimally 1.

For both the hinge region and the transmembrane region (transmembrane domain), the CAR may be designed to include a transmembrane domain fused to the extracellular domain of the CAR. In one embodiment, a transmembrane domain that is naturally associated with one of the domains in the CAR is used. In some examples, the transmembrane domain may be selected, or modified by amino acid substitutions, to avoid binding such a domain to the transmembrane domain of the same or a different surface membrane protein, thereby minimizing interaction with other members of the receptor complex.

The extracellular omains of the ECAR of the present invention comprise endogenous protein molecules, preferably endogenous protein molecules having a specific sequence.

In the present invention, the intracellular domains in the ECAR of the present invention include the transmembrane region of CD28, the co-stimulatory factor of CD28, and the signaling domain of CD3ζ.

In a preferred embodiment of the present invention, the amino acid sequence of said ECAR is shown in any one of SEQ ID NO.: 6-10.

MALPVTALLLPLALLLHAIPTEIPTSALVKETLALLSTHRTLLIANETLRIP
VPVHKNHQLCTEEIFQGIGTLESQTVQGGTVERLFKNLSLIKKYIDGQKKKCG
EERRRVNQFLDYLQEFLGVMNTEWIIESRAAAIEVMYPPPYLDNEKSNGTIIH
VKGKHLCPSPLFPGPSKPFWVLVVVGGVLACYSLLVTVAFIIFWVRSKRSRL
LHSDYMNMTPRRPGPTRKHYQPYAPPRDFAAYRSRVKFSRSADAPAYQQGQ
NQLYNELNLGRREEYDVLDKRRGRDPEMGGKPRRKNPQEGLYNELQKDKM
AEAYSEIGMKGERRRGKGHDGLYQGLSTATKDTYDALHMQALPPR (SEQ ID
NO.: 6, endogenous chimeric antigen receptor CAR (ECAR) hCD28 signaling
peptide-hIL5-hCD28 hinge region-hCD28 transmembrane region-hCD28 co-
stimulatory molecule-CD3ζ cytoplasmic signaling sequence, hIL5-ECAR)

MASPLTRFLSLNLLLLGESIILGSGEAMEIPMSTVVKETLTQLSAHRALL
TSNETMRLPVPTHKNHQLCIGEIFQGLDILKNQTVRGGTVEMLFQNLSLIKKY
IDRQKEKCGEERRRTRQFLDYLQEFLGVMSTEWAMEGRAAASTTTKPVLRT
PSPVHPTGTSQPQRPEDCRPRGSVKGTGLDFACDIYIWAPLAGICVALLLSLII
TLICYNSRRNRLLQSDYMNMTPRRPGLTRKPYQPYAPARDFAAYRPRAKFSR
SAETAANLQDPNQLYNELNLGRREEYDVLEKKRARDPEMGGKQQRRRNPQ
EGVYNALQKDKMAEAYSEIGTKGERRRGKGHDGLYQGLSTATKDTYDALH
MQTLAPRGSG (SEQ ID NO.:7, mCD8α signaling peptide-mIL5-mCD8α hinge
region-mCD8α transmembrane region-mCD28 co-stimulatory molecule-CD3ζ
cytoplasmic signaling sequence, mIL5-ECAR)

MALPVTALLLPLALLLHAYPYDVPDYASNELHQVPSNCDCLNGGTCVS
NKYFSNIHWCNCPKKFGGQHCEIDKSKTCYEGNGHFYRGKASTDTMGRPCL
PWNSATVLQQTYHAHRSDALQLGLGKHNYCRNPDNRRRPWCYVQVGLKLL
VQECMVHDCADGKKPSSPPEELKFQCGQKTLRPRFKIIGGEFTTIENQPWFAA
IYRRHRGGSVTYVCGGSLISPCWVISATHCFIDYPKKEDYIVYLGRSRLNSNT
QGEMKFEVENLILHKDYSADTLAHHNDIALLKIRSKEGRCAQPSRTIQTICLP
SMYNDPQFGTSCEITGFGKENSTDYLYPEQLKMTVVKLISHRECQQPHYYGS
EVTTKMLCAADPQWKTDSCQGDSGGPLVCSLQGRMTLTGIVSWGRGCALK
DKPGVYTRVSHFLPWIRSHTKEENGLALRAAAIEVMYPPPYLDNEKSNGTIIH
VKGKHLCPSPLFPGPSKPFWVLVVVGGVLACYSLLVTVAFIIFWVRSKRSRL
LHSDYMNMTPRRPGPTRKHYQPYAPPRDFAAYRSRVKFSRSADAPAYQQGQ
NQLYNELNLGRREEYDVLDKRRGRDPEMGGKPRRKNPQEGLYNELQKDKM
AEAYSEIGMKGERRRGKGHDGLYQGLSTATKDTYDALHMQALPPR (SEQ ID

NO.:8, hCD28 signaling peptide-hPlau-hCD28 hinge region-hCD28 transmembrane
region-hCD28 co-stimulatory molecule-CD3ζ cytoplasmic signaling sequence,
hPLau-ECAR)

MALPVTALLLPLALLLHAYPYDVPDYAGPASVPTTCCFNLANRKIPLQR
LESYRRITSGKCPQKAVIFKTKLAKDICADPKKKWVQDSMKYLDQKSPTPKP
RAAAIEVMYPPPYLDNEKSNGTIIHVKGKHLCPSPLFPGPSKPFWVLVVVGG
VLACYSLLVTVAFIIFWVRSKRSRLLHSDYMNMTPRRPGPTRKHYQPYAPPR
DFAAYRSRVKFSRSADAPAYQQGQNQLYNELNLGRREEYDVLDKRRGRDPE
MGGKPRRKNPQEGLYNELQKDKMAEAYSEIGMKGERRRGKGHDGLYQGLS
TATKDTYDALHMQALPPR (SEQ ID NO.:9, hCD28 signaling peptide-hCCL11-
hCD28 hinge region-hCD28 transmembrane region-hCD28 co-stimulatory molecule-
CD3ζ cytoplasmic signaling sequence, hCCL11-ECAR)

MALPVTALLLPLALLLHAYPYDVPDYAVVIPSPCCMFFVSKRIPENRVV
SYQLSSRSTCLKAGVIFTTKKGQQFCGDPKQEWVQRYMKNLDAKQKKASPR
ARAVAVKGPVQRYPGNQTTCRAAAIEVMYPPPYLDNEKSNGTIIHVKGKHL
CPSPLFPGPSKPFWVLVVVGGVLACYSLLVTVAFIIFWVRSKRSRLLHSDYM
NMTPRRPGPTRKHYQPYAPPRDFAAYRSRVKFSRSADAPAYQQGQNQLYNE
LNLGRREEYDVLDKRRGRDPEMGGKPRRKNPQEGLYNELQKDKMAEAYSE
IGMKGERRRGKGHDGLYQGLSTATKDTYDALHMQALPPR (SEQ ID NO.:10,
hCD28 signaling peptide-hCCL24-hCD28 hinge region-hCD28 transmembrane
region-hCD28 co-stimulatory molecule-CD3ζ cytoplasmic signaling sequence,
hCCL24-ECAR)

In a preferred embodiment of the present invention, the nucleotide sequence of said ECAR is as shown in any one of SEQ ID NO.: 11-15.
wherein positions 1-54 in SEQ ID NO.:6 are signal peptides; positions 55-399 are endogenous protein molecules (e.g., human IL5); positions 412-528 are hinge regions (e.g., the hinge region of human CD28); positions 529- 609 are transmembrane regions (e.g., transmembrane region of human CD28); positions 610-732 are co-stimulatory elements (e.g., co-stimulatory elements of human CD28); and positions 733-1068 are CD3ζ.

Of these, positions 1-81 in SEQ ID NO.:7 are signal peptides; positions 82-420 are endogenous protein molecules (e.g., murine IL5); positions 433-570 are hinge regions (e.g., hinge region of murine CD8α); positions 571 -633 positions are transmembrane regions (e.g., transmembrane region of murine CD8α); positions 640-762 are co-stimulatory elements (e.g., co-stimulatory elements of murine CD28); and positions 763-1101 are CD3ζ.

Of these, positions 1-54 in SEQ ID NO.:8 are signal peptides; positions 82-1314 are endogenous protein molecules (e.g., human Plau); positions 1327-1443 are hinge regions (e.g., hinge regions of human CD28); positions 1444 -1524 positions are transmembrane regions (e.g., transmembrane region of human CD28); positions 1525-1647 are co-stimulatory elements (e.g., co-stimulatory elements of human CD28); and positions 1648-1983 are CD3ζ.

Of these, positions 1-54 in SEQ ID NO.:9 are signal peptides; positions 82-303 are endogenous protein molecules (e.g., human CCL11); positions 316-432 are hinge regions (e.g., hinge region of human CD28); positions 433 -513 positions are transmembrane regions (e.g., transmembrane region of human CD28); positions 514-636 are co-stimulatory elements (e.g., co-stimulatory elements of human CD28); and positions 637-972 are CD3ζ.
wherein positions 1-54 in SEQ ID NO.: 10 are signal peptides; positions 82-360 are endogenous protein molecules (e.g., human CCL24); positions 373-489 are hinge regions (e.g., hinge region of human CD28); positions 490 -570 positions are transmembrane regions (e.g., transmembrane region of human CD28); positions 571-693 are co-stimulatory elements (e.g., co-stimulatory elements of human CD28); positions 694-1029 are CD3ζ.

### Chimeric antigen receptor T cells (CAR-T cells)

As used herein, the terms "CAR-T cell", "CAR-T", "CAR-T cell of the present invention", "CAR-T cell of the present invention", "antigen receptor T cell", "ECAR-T cell", "ECAR-T", "ECAR-T cell of the present invention", "ECAR-T cell of the present invention", and "endogenous antigen receptor T cell" all refer to ECAR-T cells as described in the sixth aspect of the present invention, wherein the ECAR-T cells of the present invention can be targeted to receptors corresponding (matching) to endogenous protein molecules and are used to kill or inactivate cells (e.g., inflammatory cells, senescent cells, tumor cells, autoimmune cells) or to treat diseases (e.g., neoplastic diseases, allergic diseases, autoimmune diseases, senescent cell-associated diseases).

CAR-T cells have the following advantages over other T-cell-based therapeutic modes: (1) the course of action of CAR-T cells is not restricted by MHC; (2) given that many cells express the same antigens, CAR gene constructs targeting a particular antigen, once completed, can be widely utilized; (3) CARs can utilize both protein antigens as well as glycolipid-based non-protein antigens, expanding the the range of antigen targets; (4) the use of patient autologous cells reduces the risk of rejection; and (5) the CAR-T cells have an immune memory function and can survive in the body for a long time.

In the present invention, the CAR of the present invention comprises (i) an extracellular domain, which is an endogenous protein molecule; (ii) optionally a hinge region; (iii) a transmembrane domain; (iv) a co-stimulatory factor; and (v) a signaling domain of CD3ζ.

### Chimeric antigen receptor NK cells (CAR-NK cells)

As used herein, the term "CAR-NK cell", "CAR-NK", "CAR-NK cell of the present invention", "CAR-NK cell of the present invention", "antigen receptor NK cell", "ECAR-NK cell", "ECAR-NK", "ECAR-NK cell of the present invention", "ECAR-NK cell of the present invention", and "endogenous antigen receptor NK cell" all refer to ECAR-NK cells described in the first aspect of the present invention. The CAR-NK cells of the present invention can be targeted to receptors corresponding (matching) to endogenous protein molecules for killing cells (e.g., inflammatory cells, senescent cells, tumor cells, autoimmune cells).

Natural killer (NK) cells are a major class of immune effector cells that protect against viral infections and tumor cells through non-antigen-specific pathways. Engineered (genetically modified) NK cells may acquire new functions, including the ability to specifically recognize cellular antigens and to have enhanced cell killing effects.

CAR-NK cells also have advantages over autologous CAR-T cells, such as: (1) they kill cells directly by releasing perforin and granzyme without killing normal cells in the body; (2) they release a very small amount of cytokines, thus reducing the risk of a cytokine storm; and (3) they are very easy to expand in vitro and to develop into "off-the-shelf' products. Otherwise, similar to CAR-T cell therapy.

### Chimeric Antigen Receptor Macrophages (CAR-Macrophages)

As used herein, the terms "CAR-macrophage", "CAR-macrophage of the present invention", "CAR-macrophage of the present invention", "antigen receptor macrophage", "ECAR-macrophage", "ECAR-macrophage of the present invention", "ECAR-macrophage of the present invention", "endogenous antigen receptor macrophage" refer to ECAR-macrophage as described in the first aspect of the present invention. The CAR-macrophages of the present invention can be targeted to receptors matching endogenous protein molecules and used to kill or sterilize cells (e.g., inflammatory cells, senescent cells, tumor cells, autoimmune cells) or to treat disease (e.g., inflammatory cells, senescent cells, tumor cells, autoimmune cells).

### Exogenous T cell antigen receptor

As used herein, exogenous T cell antigen receptor (T cell receptor, TCR) is the α and β chains of the TCR cloned from tumor-reactive T cells by gene transfer techniques, by means of genetic engineering, lentiviruses or retroviruses are used as vectors and exogenously transferred to the TCR in T cells.

Exogenous TCR-modified T cells are capable of specifically recognizing and killing cells, and by optimizing the affinity of the TCR for specific antigens, the affinity of the T cell for the cell to be killed can be increased, improving the effect of killing the cell.

### Vectors

Nucleic acid sequences encoding the desired molecule may be obtained using recombinant methods known in the art, such as, for example, by screening libraries from cells expressing the gene, by obtaining the gene from vectors known to include the gene, or by direct isolation of the gene from cells and tissues comprising the gene using standard techniques. Optionally, the gene of interest may be produced synthetically.

The present invention also provides vectors in which the expression cassettes of the present invention are inserted. Vectors derived from retroviruses such as lentiviruses are suitable tools for achieving long-term gene transfer, as they allow for long-term, stable integration of the transgene and its proliferation in daughter cells. Lentiviral vectors have advantages over vectors derived from oncogenic retroviruses such as murine leukemia virus because they can transduce non-proliferating cells such as hepatocytes. They also have the advantage of low immunogenicity.

In brief summary, it is generally operable to ligate an expression cassette or nucleic acid sequence of the invention to a promoter and incorporate it into an expression vector. The vectors are suitable for replication and integration into eukaryotic cells. Typical cloning vectors contain transcriptional and translational terminators, initial sequences and promoters that can be used to regulate the expression of desired nucleic acid sequences.

The expression constructs of the present invention may also be utilized in standard gene delivery schemes for nucleic acid immunization and gene therapy. Methods of gene delivery are known in the art. See, e.g., U.S. Patent Nos. 5,399,346, 5,580,859, 5,589,466, which are hereby incorporated by reference in their entirety. In another embodiment, the present invention provides gene therapy vectors.

The nucleic acid may be cloned into many types of vectors. For example, the nucleic acid may be cloned into so vectors, which include, but are not limited to, plasmids, phages, phage derivatives, animal viruses, and mucoids. Specific vectors of interest include expression vectors, replication vectors, probe generation vectors, and sequencing vectors.

Further, the expression vectors may be provided to the cells in the form of viral vectors. Viral vector technology is well known in the art and described in, for example, Sambrook et al. (2001, Molecular Cloning: A Laboratory Manual, Cold Spring Harbor Laboratory, New York) and other virology and molecular biology manuals. Viruses that may be used as vectors include, but are not limited to, retroviruses, adenoviruses, adeno-concomitant viruses, herpesviruses, and lentiviruses. Typically, suitable vectors comprise a replication start that functions in at least one organism, a promoter sequence, a convenient restriction enzyme site, and one or more selectable markers (e.g., WO01/96584; WO01/29058; and U.S. Patent No. 6,326,193).

Many virus-based systems have been developed for gene transfer into mammalian cells. For example, retroviruses provide a convenient platform for use in gene delivery systems. Selected genes can be inserted into a vector and packaged into retroviral particles using techniques known in the art. The recombinant virus may then be isolated and delivered to the subject cell in vivo or in vitro. Many retroviral systems are known in the art. In some embodiments, an adenoviral vector is used. Many adenoviral vectors are known in the art. In one embodiment, a lentiviral vector is used.

Additional promoter elements, such as enhancers, can regulate the frequency of transcription initiation. Typically, these are located in a 30-110 bp region upstream of the start site, although it has recently been shown that many promoters also contain functional elements downstream of the start site. The spacing between promoter elements is often flexible to maintain promoter function when the element is inverted or moved relative to another. In the thymidine kinase (tk) promoter, the spacing between promoter elements can be increased by 50 bp apart before activity begins to decline. Depending on the promoter, exhibit individual elements can act cooperatively or independently to initiate transcription.

An example of a suitable promoter is the immediate early cytomegalovirus (CMV) promoter sequence. This promoter sequence is a strong constitutive promoter sequence capable of driving high level expression of any polynucleotide sequence operably linked thereto. Another example of a suitable promoter is elongation growth factor-1α (EF-1α). However, other constitutive promoter sequences may also be used including, but not limited to, simian virus 40 (SV40) early promoter, mouse mammary carcinoma virus (MMTV), human immunodeficiency virus (HIV) long terminal repeat (LTR) promoter, MoMuLV promoter, ornithosis leukemia virus promoter, Epstein-Barr virus immediate early promoter, Rousse's sarcoma virus promoter, and human gene promoters such as, but not limited to, actin promoters, myosin promoters, heme promoters, and creatine kinase promoters. Further, the present invention should not be limited to the application of constitutive promoters. Inducible promoters are also contemplated as part of the present invention. The use of inducible promoters provides molecular switches that are capable of turning on expression of polynucleotide sequences operably linked to an inducible promoter when such expression is desired, or turning off expression when expression is undesired. Examples of inducible promoters include, but are not limited to, metallothionein promoters, glucocorticoid promoters, progesterone promoters, and tetracycline promoters.

In order to assess expression of the CAR polypeptide or portion thereof, the expression vector that is introduced into the cell may also comprise either or both of selectable marker genes or reporter genes to facilitate identification and selection of expressing cells from a population of cells seeking to be transfected or infected via a viral vector. In other aspects, the selectable marker may be carried on a separate segment of DNA and used in a cotransfection program. Both the selectable marker and the reporter gene may be flanked by appropriate regulatory sequences to enable expression in the host cell. Useful selectable markers include, for example, antibiotic resistance genes such as neo and the like.

The reporter gene is used to identify potentially transfected cells and for evaluating the functionality of the regulatory sequence. Typically, the reporter gene is a gene which is not present in or expressed by the recipient organism or tissue and which encodes a polypeptide, the expression of which is clearly indicated by some readily detectable property such as enzymatic activity. The expression of the reporter gene is measured at a suitable time after the DNA has been introduced into the recipient cell. Suitable reporter genes may include genes encoding luciferase, β-galactosidase, chloramphenicol acetyltransferase, secreted alkaline phosphatase, or green fluorescent protein (e.g., Ui-Tei et al., 2000 FEBS Letters 479:79-82). Suitable expression systems are well known and can be prepared using known techniques or are commercially available. Typically, constructs with a minimum of five flanking regions that show the highest level of reporter gene expression are identified as promoters. Such promoter regions can be ligated to the reporter gene and used to evaluate the ability of the reagent to regulate promoter-driven transcription.

Methods of introducing genes into cells and expressing genes into cells are known in the art. In the content of expression vectors, the vector may be readily introduced into a host cell, e.g., a mammalian, bacterial, yeast, or insect cell, by any method in the art. For example, the expression vector may be transferred into the host cell by physical, chemical or biological means.

Physical methods for introducing a polynucleotide into a host cell include calcium phosphate precipitation, lipid transfection methods, particle bombardment, microinjection, electroporation, and the like. Methods for producing cells comprising vectors and/or exogenous nucleic acids are well known in the art. See, for example, Sambrook et al. (2001, Molecular Cloning: a Laboratory Manual, Cold Spring Harbor Laboratory, New York). A preferred method for introducing polynucleotides into host cells is calcium phosphate transfection.

Biological methods for introducing polynucleotides of interest into host cells include the use of DNA and RNA vectors. Viral vectors, particularly retroviral vectors, have become the most widely used method of inserting genes into mammalian, e.g. human, cells. Other viral vectors can be derived from lentiviruses, poxviruses, herpes simplex virus I, adenoviruses and adeno-associated viruses, among others. See, e.g., U.S. Patent Nos. 5,350,674 and 5,585,362.

Chemical means of introducing polynucleotides into host cells include colloidal dispersion systems, such as macromolecular complexes, nanocapsules, microspheres, beads; and lipid-based systems, including oil-in-water emulsions, micelles, hybrid micelles, and liposomes. Exemplary colloidal systems used as in vitro and in vivo delivery vehicles are liposomes (e.g., artificial membrane vesicles).

In the case of using non-viral delivery systems, exemplary delivery vehicles are liposomes. The use of a lipid formulation is contemplated for introducing a nucleic acid into a host cell (in vitro, ex vivo (isolated), or in vivo). In another aspect, the nucleic acid may be associated with a lipid. The nucleic acid associated with a lipid may be encapsulated into the aqueous interior of a liposome, dispersed within the lipid bilayer of a liposome, attached to the liposome by a linker molecule associated with both the liposome and the oligonucleotide, trapped in the liposome, complexed with the liposome, dispersed in a solution comprising the lipid, mixed with the lipid, associated with the lipid, contained in the lipid as a suspension, contained in or complexed with micelles, or otherwise associated with the lipid. manner associated with the lipid. The lipids, lipids/DNA, or lipids/expression vectors associated with the compositions are not limited to any specific structure in solution. For example, they may be present in bilayer structures, as micelles or have a "collapsed" structure. They may also simply be dispersed in solution and may form aggregates of uneven size or shape. Lipids are fatty substances, which may be naturally occurring or synthetic. For example, lipids include fat droplets, which occur naturally in the cytoplasm as well as in compounds of the class comprising long chain aliphatic hydrocarbons and their derivatives such as fatty acids, alcohols, amines, amino alcohols and aldehydes.

In a preferred embodiment of the invention, said vector is a lentiviral vector.

### preparations

The present invention provides a CAR as described in the first aspect of the present invention, a nucleic acid molecule as described in the second aspect of the present invention, a vector as described in the third aspect of the present invention, or a host cell as described in the fourth aspect of the present invention, as well as a pharmaceutically acceptable carrier, diluent or excipient. In one embodiment, said formulation is a liquid formulation. Preferably, said formulation is an injection. Preferably, the concentration of said CAR-T cells in said formulation is 1×10⁵-1×10⁸ cells/mL, preferably 1×10⁶-1×10⁷ cells/mL , better still 1 ×10⁶-5×10⁶ cells/mL. in one embodiment, said formulation may comprise a buffer such as neutral buffered saline, sulfate buffered saline, and the like; carbohydrates such as glucose, mannose, sucrose or dextran, mannitol; proteins; peptides or amino acids such as glycine; antioxidants; chelating agents such as EDTA or glutathione; adjuvants (e.g., aluminum hydroxide); and preservatives. The formulations of the present invention are preferably formulated for intra-venous administration.

### Therapeutic Applications

The present invention includes therapeutic applications with cells (e.g., T cells) transduced with lentiviral vectors (LVs) encoding expression cassettes of the present invention. The transduced T cells can target receptors corresponding (matching) to endogenous protein molecules, synergistically activating the T cells and eliciting a cellular immune response, thereby significantly increasing their cell killing or killing efficiency.

Accordingly, the present invention also provides methods of stimulating a T cell - mediated immune response to a target cell population or tissue of a mammal comprising the steps of: administering to the mammal a CAR-T cell of the present invention.

In one embodiment, the present invention comprises a class of cell therapies in which patient autologous T cells (or heterologous donors) are isolated, activated and genetically modified to produce ECAR-T cells, which are subsequently injected into the same patient. This approach has a very low probability of developing graft-versus-host disease, and the antigen is recognized by the T-cells in an MHC-restriction-free manner. In addition, one ECAR-T can treat all diseases that express receptors corresponding to that endogenous protein molecule. Unlike antibody therapies, ECAR-T cells are capable of replicating in vivo, producing long-term persistence that can lead to sustained cell killing or killing control.

In one embodiment, the ECAR-T cells of the present invention may undergo solid in vivo T cell expansion and may be sustained for an extended amount of time. Alternatively, the ECAR-mediated immune response may be part of a step of overt immunotherapy, wherein the ECAR-modified T cells and induce a specific immune response of the T cells to the target cells.

Although the data disclosed herein specifically discloses lentiviral vectors comprising endogenous protein molecules, hinge and transmembrane regions, and CD28 and CD3ζ signaling structural domains, the present invention should be construed to encompass any number of variations to each of the construct components.

Diseases that may be treated include oncological diseases, allergic diseases, autoimmune diseases, and senescent cell-associated diseases.

The CAR-modified T cells of the present invention may also be used as a vaccine type for ex vivo immunization and/or in vivo therapy in mammals. Preferably, the mammal is human.

For ex vivo immunization, at least one of the following occurs in vitro prior to administration of the cells into the mammal: i) expansion of the cells, ii) introduction of nucleic acid encoding the CAR into the cells, and/or iii) cryopreservation of the cells.

The ex vivo procedure is well known in the art and is discussed more fully below. Briefly, cells are isolated from a mammal (preferably a human) and genetically modified (i.e., transduced or transfected in vitro) with a vector expressing a CAR disclosed herein. The CAR-modified cells may be administered to a mammalian recipient to provide a therapeutic benefit. The mammalian recipient may be human, and the CAR-modified cells may be autologous relative to the recipient. Optionally, the cells may be homozygous, homozygous (syngeneic), or heterozygous relative to the recipient.

In addition to the use of cell-based vaccines with respect to ex vivo immunization, the present invention also provides compositions and methods for in vivo immunization to elicit an immune response against an antigen in a patient.

The present invention provides methods of treating a disease comprising administering to a subject in need thereof a therapeutically effective amount of a CAR-modified T cell of the present invention.

The CAR-modified T cells of the present invention may be administered alone or as a pharmaceutical composition in combination with a diluent and/or with other components or other cytokines or cell populations. Briefly, the pharmaceutical compositions of the present invention may include target cell populations as described herein, in combination with one or more pharmaceutically or physiologically acceptable carriers, diluents or excipients. Such compositions may include buffers such as neutral buffered saline, sulfate buffered saline, and the like; carbohydrates such as glucose, mannose, sucrose or dextran, mannitol; proteins; peptides or amino acids such as glycine; antioxidants; chelating agents such as EDTA or glutathione; adjuvants (e.g., aluminum hydroxide); and preservatives. The compositions of the present invention are preferably formulated for intravesical administration.

The pharmaceutical compositions of the present invention may be administered in a manner suitable for the disease to be treated (or prevented). The amount and frequency of administration will be determined by such factors as the patient's condition, and the type and severity of the patient's disease - although the appropriate dosage may be determined by clinical trials.

When "immunologically effective amount" or "therapeutic amount" is indicated, the precise amount of the composition of the present invention to be administered may be determined by a physician taking into account individual differences in the patient's (subject's) age, weight, degree of infection or metastasis, and disease. It may be generally noted that: pharmaceutical compositions comprising the T cells described herein may be administered at a dose of 104 to 109 cells/kg of body weight, preferably 105 to 106 cells/kg of body weight (including all integer values within those ranges). the T cell compositions may also be administered multiple times at these doses. The cells may be administered by using injection techniques well known in immunotherapy (see, e.g., Rosenberg et al, NewEng. J. of Med. 319:1676, 1988). The optimal dosage and treatment regimen for a particular patient can be readily determined by a person skilled in the art of medicine by monitoring the patient for signs of disease and thus modulating the treatment.

Administration of the subject compositions may be carried out in any convenient manner, including by spray method, injection, swallowing, infusion, implantation or transplantation. The compositions described herein may be administered to a patient subcutaneously, intradermally, intratumorally, intranodally, intraspinal, intramuscularly, by intravenously (i.v.) injection, or intraperitoneally. In one embodiment, the T cell compositions of the present invention are administered to a patient by intradermal or subcutaneous injection. In another embodiment, the T cell compositions of the present invention are preferably administered by i.v. injection. the compositions of T cells may be injected directly into the cell to be killed, a tumor, a lymph node, or a site of infection.

In certain embodiments of the present invention, cells activated and expanded using the methods described herein or other methods known in the art for expanding T cells to therapeutic levels are administered to a patient in combination with (e.g., prior to, concurrently with, or subsequent to) any number of related therapeutic forms, said therapeutic forms comprising, but not limited to, treatment with the following reagents: said reagents such as antiviral therapy, cidofovir, and leukocyte interleukin-2, cytarabine (also known as ARA-C) or natalizumab therapy for MS patients or erfalizumab therapy for psoriasis patients or other therapies for PML patients. In further embodiments, the T cells of the present invention may be used in combination with: chemotherapy, radiation, immunosuppressive agents such as, for example, cyclosporine, azathioprine, methotrexate, meclofenoxate and FK506, antibodies or other immunotherapeutic agents. In further embodiments, the cellular compositions of the present invention are administered to a patient in combination (e.g., before, at the same time, or after) with a bone marrow transplant, utilizing a chemotherapeutic agent such as fludarabine, external beam radiation therapy (XRT), cyclophosphamide. For example, in one embodiment, the subject may undergo standard treatment with high-dose chemotherapy followed by peripheral blood stem cell transplantation. In some embodiments, following the transplant, the subject receives an infusion of the expanded immune cells of the present invention. In an additional embodiment, the expanded cells are administered before or after the surgical procedure.

The dosage of the above treatments administered to a patient will vary with the precise properties of the condition being treated and the recipient of the treatment. The ratio of doses administered to a person may be implemented according to practices accepted in the art. Typically, from 1 × 10⁶ to 1 × 10¹⁰ modified T cells of the present invention may be administered to a patient per treatment or per course of therapy, by, for example, intravenous infusion.

### The main advantages of the present invention include:

(1) The present invention finds for the first time that endogenous protein molecules are used as the extracellular antigen-binding structural domains of CAR instead of antibody single-domain fragments, and the endogenous chimeric antigen receptor (endogenous CAR, ECAR) is designed to kill a variety of cells specifically and selectively, and to have a significant killing effect.
(2) For the first time, the present invention uses an endogenous protein molecule as the extracellular binding domain of a chimeric antigen receptor to construct a new endogenous chimeric antigen receptor (eCAR), whose gene sequence of the extracellular structural domain is selected from a chain of protein molecules existing in the human body, and thus has a good tolerance by avoiding rejection reaction in the human body and well tolerated in the human body.
(3) The present invention designs endogenous chimeric antigen receptors (endogenous CARs) by targeting antigens that have been found to have ligands in the body, and replacing antibody single-domain fragments with endogenous protein molecules as the extracellular antigen-binding structural domains of the CARs. The method of the present invention greatly reduces the time cost, shortens the economic cost, and avoids the process of antibody preparation and screening.
(4) The endogenous chimeric antigen receptor designed by the present invention consists entirely of endogenous protein molecular chains, and therefore, unlike conventional chimeric antigen receptors carrying exogenous single structural domain antibodies, will not cause a rejection reaction in the human body.

The present invention is further described below in connection with specific embodiments. It should be understood that these embodiments are used only to illustrate the invention and are not intended to limit the scope of the invention. Experimental methods for which specific conditions are not indicated in the following embodiments are generally in accordance with conventional conditions, such as those described in Sambrook et al, Molecular Cloning: a Laboratory Manual (New York: Cold Spring Harbor Laboratory Press, 1989), or in accordance with conditions recommended by the manufacturer. Unless otherwise indicated, percentages and parts are weight percentages and weight parts.

Materials and reagents used in embodiments of the present invention are commercially available unless otherwise indicated.

### Example 1: Confirmation of hCD8α signal peptide-hIL5 (endogenous protein molecule)-hCD28 hinge region-hCD28 transmembrane region-hCD28 intracellular domain (co-stimulatory molecule)-hCD3ζ gene sequence and lentiviral vector construction

The gene sequences of human CD8α signal peptide, human IL5, human CD28 hinge region, human CD28 transmembrane region, human CD28 intracellular domain and human CD3ζ intracellular region were retrieved from NCBI database.

Qingke Biotechnology Co., LTD was commissioned to synthesize the complete hIL5-ECAR gene sequence in the order of human CD8α signal peptide gene coding region sequence, human IL5 gene coding region sequence, human CD28 hinge region gene coding region sequence, human CD28 transmembrane region gene coding region sequence, human CD28 intracellular domain gene coding region sequence as well as the gene coding region sequence of the intracellular region of human CD3ζ and hIL5-ECAR was ligated to the PHAGE lentiviral plasmid by PCR and the Non-Ligase-Dependent Single Fragment Rapid Cloning Kit from Vazyme Biotech Co.,Ltd. The ligation product was transformed into the competent (DH5α, Tsingke Biotech Co., Ltd.), the plate was coated overnight, the single clone was picked and sent to Tsingke Biotech Co., Ltd. for sequencing, and the results of the sequencing results were compared to confirm whether the plasmid was constructed successfully or not.

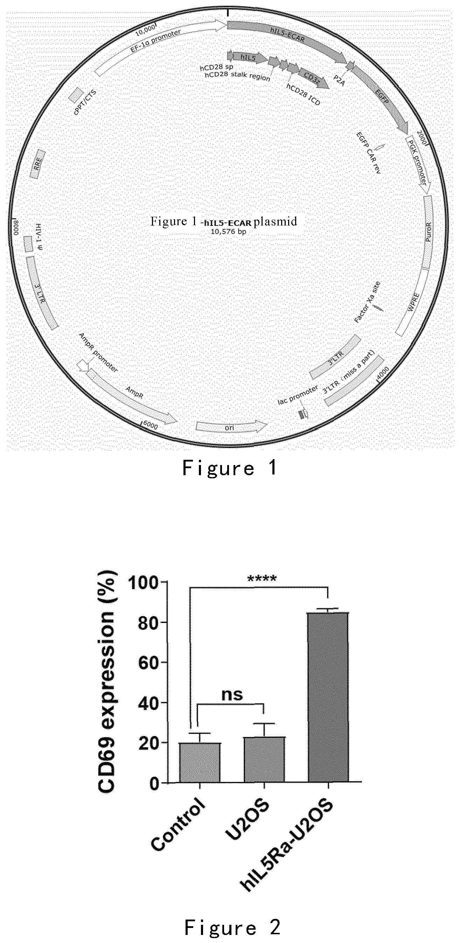

The purified hIL5-ECAR lentiviral plasmid was extracted using the Plasmid Macro Extraction Kit from Cowin Biotech Co., Ltd. (an example of the plasmid map is shown in Figure 1).

### Example 2: hIL5-ECAR lentivirus packaging and concentration

The hIL5-ECAR plasmid obtained in Example 1 was used to package the virus by transfection of 293T using the PEI method, and the virus was concentrated by ultracentrifugation as follows:
Day 1: 293T cells with less than 20 generations and a cell density of about 90% were selected and plated at a cell density of 30% in a 15-cm dish containing 30 mL of DMEM complete medium, the cells were well mixed, and cultured overnight at 37°C in a 5% CO2 incubator.
Day 2: Observe that the 293T cell density reaches 70%-90%, discard the medium, add 27mL of fresh DMEM medium containing 10% serum, and prepare for transfection. Prepare plasmid mixture: Add 1500 µL of DMEM medium to a clean 15 mL centrifuge tube, and add 22.5 µg of hIL5-ECAR lentiviral plasmid obtained in Example 1, 16.875 µg of psPAX2, and 5.625 µg of pMD2.G, and mix thoroughly; take another clean 15 mL centrifuge tube, add 1500 µL of DMEM medium and add PEI 40000 transfection reagent 112.5µL from Shanghai Yisheng Bio-Technology Co., Ltd and mix well; drop the PEI mixture into the plasmid mixture, mix well and leave it for 20min; add 3mL of the above mixture along the wall of the dish into 293T cell dish and incubate it at 37°C for 8h, discard the medium and gently refill 30mL of pre-warmed, fresh DMEM complete medium containing 10% serum along the wall of the petri dish.
Day 4: Viral supernatants were collected 36-48h after transfection and filtered through a 0.22µm filter. The filtered supernatant was transferred to an ultracentrifuge tube and flattened, and centrifuged in an ultracentrifuge for 90 min at 4°C at 35,000 rpm. At the end of centrifugation, the supernatant was aspirated and discarded, the precipitate obtained from each 30 mL of viral stock solution was resuspended with 300 µL of DMEM medium and the resulting concentrated hIL5-ECAR lentiviral solution was used for infection within 24 h or stored at -80°C.

### Example 3: CD69 expression assay after co-culture of hIL5-ECAR Jurkat with hIL5Ra target cells

The hIL5-ECAR virus concentrated in Example 2 was infected with the Jurkat cell line to obtain the hIL5-ECAR Jurkat stably transfected cell line. The experimental group contained 1 ×10⁵ hIL5-ECAR Jurkat cells and 1 ×10⁵ hIL5Ra target cells (U₂OS cell line) per well; the negative control group contained 1 ×10⁵ hIL5-ECAR Jurkat cells and 1 ×10⁵ target cells without hIL5Ra (U₂OS cell line) per well; and the blank group contained only 1×10⁵ hIL5-ECAR Jurkat cells per well. The total system was 200 µL of 1640 complete medium containing 10% serum, and the two types of cells were mixed thoroughly and then added to 96-well plates for co-culture.

The cells were collected after 24 h of incubation in the incubator, and each tube of cells was washed once with 1 mL of PBS (Beijing Solarbio Science & Technology Co., Ltd), centrifuged at 400 g for 5 min, and the supernatant was removed. Add 100 µL PBS and 0.5 µL Biolegend's human CD69 flow antibody (PE fluorescent), stain for 30 min at 4°C, avoiding light. Each tube of cells was washed once with 1 mL of 1 ×PBS, centrifuged at 400g for 5 min, and supernatant was removed. 200 µL PBS was added and resuspended, and the expression of CD69 on the surface of hIL5-ECAR Jurkat cells was detected using flow cytometry.

Figure 2 shows significant activation of hIL5-ECAR Jurkat by hIL5Ra target cells (mimicking inflammatory cells (eosinophilic granulocyte)) after co-culture.

### Example 4: Purification and culture of human T cells

Human peripheral blood mononuclear cells were obtained using DAYOU's Lymphatic Isolation Solution, and CD3 T lymphocytes were isolated and purified using Biolegend's CD3 T Lymphocyte Negative Selection Kit. Cell density was adjusted to 1×10⁶/mL with X-VIVO 15 medium from LONZA, and 1×10⁶ quantity of CD3/CD28 magnetic beads (Gibco) was added to stimulate culture for 48 hours.

### Example 5: hIL5-ECAR lentivirus infection of human T cells with flow detection of viral infection efficiency

The hIL5-ECAR concentrated virus obtained in Example 3 was utilized and human T cells were infected by centrifugal infection method to prepare CART cells, and the expression of hIL5-ECAR was detected by flow cytometry as follows:
After two days of T cell activation culture, cell counting was performed and T cell density was adjusted to 1×10⁶/well, 500 µL of fresh X-VIVO 15 was added to each well and inoculated into a new 24-well plate.

Preparation of virus solution: add 100 µL of virus concentrate from Example 2, 6 µg/mL Polybrene (Shanghai Yisheng Biotechnology Co., Ltd.), and 400 µL of X-VIVO 15 medium per well.

Centrifugation of infected T cells: place the 24-well plate in a centrifuge at 32 °C, 1500g, and centrifuge for 2h.

After centrifugation, the infected T cells were centrifuged at 1600 rpm for 5 min, the supernatant was aspirated and discarded, and each well was seeded with 1 mL of fresh X-VIVO medium in a new 24-well plate. The plates were incubated at 37°C in an incubator with 5% CO2.

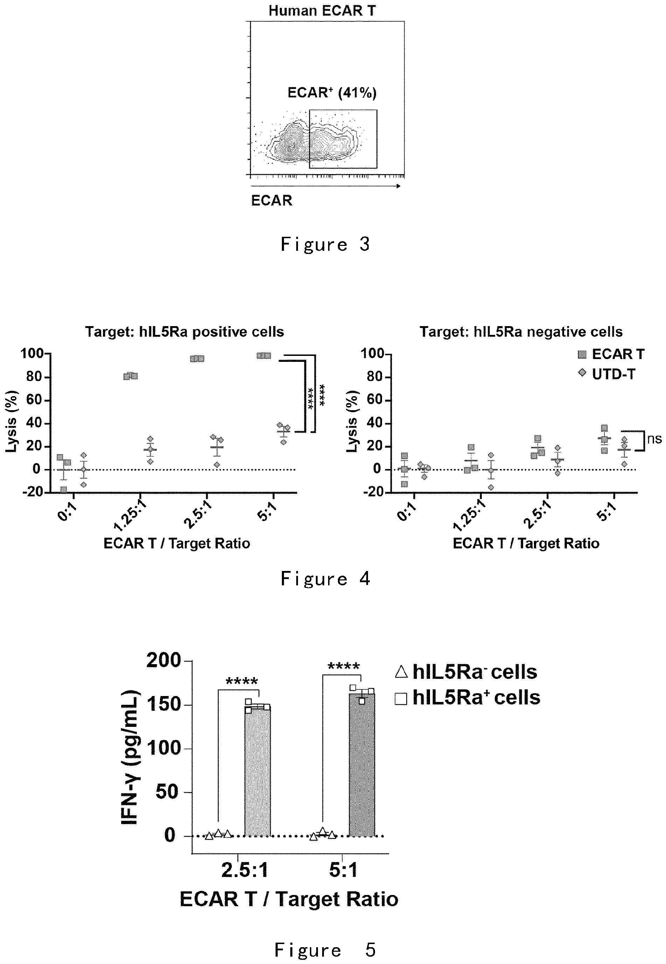

After 72h of culture, 5×10⁵ CAR T cells and T cells (control) were collected in flow tubes, respectively, and the supernatant was discarded after washing once with PBS, and the flow antibody to hIL5 from Biolegend was added to avoid light staining for 30min, followed by washing with PBS, resuspension in 200 µL of PBS, and detection by flow cytometry. The results are shown in Figure 3, the hIL5-ECAR lentivirus obtained in Example 2 has an expression efficiency of 41% of hIL5-ECAR after 72h of T cell infection.

### Example 6: Killing assay of hIL5-ECAR T after co-culture with hIL5Ra-expressing target cells (luciferase assay)

The hIL5Ra-U₂OS target cells with luciferase were constructed by lentiviral infection method, and the hIL5-ECAR T cell killing ability was detected by luciferase luminescence intensity as follows:
Prepare WHB all-white 96-well plates, resuspend hIL5Ra-U₂OS-Luciferase target cells, U2OS-Luciferase cells with X-VIVO 15 medium, adjust the cell density to 1×10⁴/well, 100 µL per well.

Resuspend the hIL5-ECAR T cells prepared in Example 5 with X-VIVO 15 medium, adjust the cell density to 2×10⁶/mL, gradient dilution and seed the plate with cell densities of 5×10⁴/well, 2.5×10⁴/well, 1.25×10⁴/well, and 0/well, 100 µL per well.

100 µL of hIL5Ra-U₂OS-Luciferase target cells, 100 µL of different cell densities of hIL5-ECAR T cells or uninfected T cells (UTD-T) were added to each well of 96-well plates in the experimental group; negative control 96-well plates were supplemented with 100 µL of U₂OS-Luciferase target cells, 100 µL of different cell densities of hIL5-ECAR T cells or uninfected T cells (UTD-T) per well. After sufficient mixing, the plates were incubated in an incubator at 37°C with 5% CO2 for 24 hours.

After 24 hours, remove the 96-well plate. The medium in the well plates was poured out, and 1 µL of coenzyme A (Beijing Solarbio Science & Technology Co., Ltd) and 1 µL of D-fluorescein (Gold Biotechnology) and 100 µL of PBS were added to each well, and protected from light for 10min, the chemiluminescence intensity was detected by using the M5 microplate reader.

The results, as shown in Figure 4, show that the killing ability of hIL5-ECAR T cells co-cultured with hIL5R-U2OS-Luciferase target cells was enhanced in a dose-dependent manner with the increase of the effector-target ratio. The cell lysis rate (Lysis) is defined as (1-(RLUₛₐₘₚₗₑ)/(RLUₘₐₓ)) × 100. RLUₛₐₘₚₗₑ is the relative light intensity of the sample detected by the microplate reader, and RLUₘₐₓ is the relative light intensity of the target cell control group without the addition of killer cells detected by the microplate reader.

### Example 7: IFN-γ secretion assay after co-culture of hIL5-ECAR T with hIL5Ra-expressing target cells

Prepare transparent 96-well plates, resuspend hIL5Ra-U₂OS target cells, U2OS cells with X-VIVO 15 medium, and adjust the cell density to 1 ×10⁴/well.

Resuspend hIL5-ECAR T cells prepared in Example 5 with X-VIVO 15 medium, adjust the cell density to 2×10⁶/mL, gradient dilution and seed the plate at a cell density of 5×10⁴/well, 2.5×10⁴/well, 100 µL per well.

100 µL of hIL5Ra-U2OS-Luciferase target cells and 100 µL of different cell densities of hIL5-ECAR T cells were added to each well of 96-well plates in the experimental group; 100 µL of U2OS-Luciferase target cells and 100 µL of different cell densities of hIL5-ECAR T cells were added to each well of 96-well plates in the negative control group. After sufficient mixing, the plates were incubated in an incubator at 37°C with 5% CO₂ for 24 hours.

After 24 hours, the 96-well plate was removed. Remove the supernatant in a 1.5mL EP tube. Centrifuge at 4°C, 400g for 5 min to remove cellular debris and retain the supernatant. The IFN-y content in the supernatant was detected using the ELISA kit for human IFN-y from Abclonal, according to the instructions.

The results are shown in FIG. 5. hIL5-ECAR T cells effectively secretes a large amount of IFN-γ after co-cultivation with hIL5R-U2OS target cells; while they failed to secrete IFN-y after co-cultivation with U2OS target cells.

### Example 8: Confirmation of mCD8α signal peptide-mIL5 (endogenous protein molecule)-mCD8a hinge region-mCD8a transmembrane region-mCD28 intracellular domain (co-stimulatory molecule)-mCD3ζ gene sequence and lentiviral vector construction

The gene sequences of mouse-derived CD8α signal peptide, mouse-derived IL5, mouse-derived CD8α hinge region, mouse-derived CD8a transmembrane region, mouse-derived CD28 intracellular domain, and mouse-derived CD3ζ intracellular region were retrieved from the NCBI database.

Tsingke Biotech Co., Ltd. was commissioned to synthesize the complete mIL5-ECAR gene sequence in the order of mouse-derived CD8α signal peptide gene coding region sequence, mouse-derived IL5 gene coding region sequence, mouse-derived CD28 hinge region gene coding region sequence, mouse-derived CD28 transmembrane region gene coding region sequence, mouse-derived CD28 intracellular domain gene coding region sequence, and the gene coding region sequence of mouse-derived CD3ζ intracellular region and mIL5-ECAR was ligated to the PMX retrovirus plasmid by PCR and the Non-Ligase-Dependent Single Fragment Rapid Cloning Kit from Vazyme Biotech Co.,Ltd. The ligation product was transformed into the competent (DH5α), the plate was coated overnight, the single clone was picked and sent to Tsingke Biotech Co., Ltd. for sequencing, and the results of the sequencing results were compared to confirm whether the plasmid was constructed successfully or not.

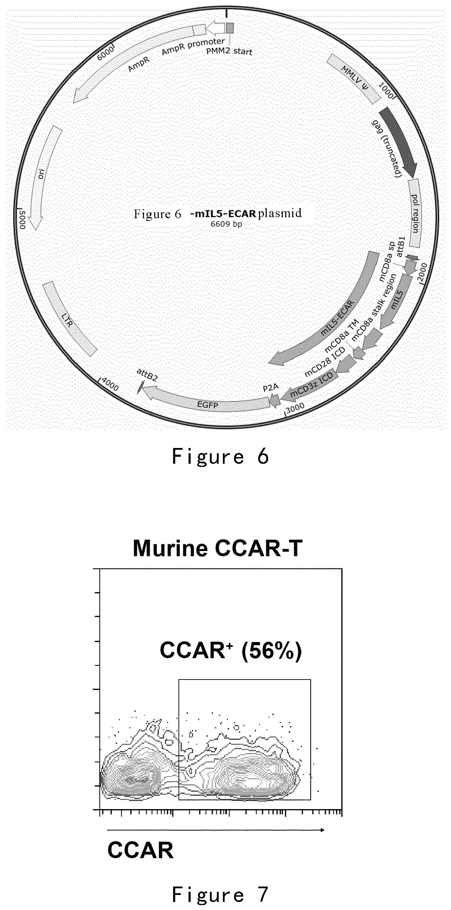

The purified mILS-ECAR lentiviral plasmid was extracted using the Plasmid Macro Extraction Kit from Cowin Biotech Co., Ltd. (an example of the plasmid map is shown in Figure 6).

### Example 9: mIL5-ECAR retrovirus packaging and concentration

The mILS-ECAR plasmid obtained in Example 8 was transfected with the Plat-E cell line using the PEI method to package the virus, and the virus was concentrated by ultracentrifugation as follows:
Day 1: Plat-E cells with less than 20 generations and a cell density of about 90% were selected and plated at a cell density of 30% in a 15-cm dish containing 30 mL of DMEM complete medium, the cells were well mixed, and cultured overnight at 37°C in a 5% CO2 incubator.
Day 2: Observe that the density of Plat-E cells reaches 70%-90%, and prepare for transfection; prepare the plasmid mixture: in a clean 15mL centrifuge tube, add 1.5mL of DMEM medium, and add 45µg of the mILS-ECAR lentiviral plasmid obtained in Example 8, and mix thoroughly; take another clean 15mL centrifuge tube, add 1.5mL of DMEM medium, and add transfection reagent PEI 112.5µL, mix well; drop the PEI mixture into the plasmid mixture, mix well, and let it stand for 20min; add 3mL of the mixture along the wall of the Petri dish to the Plat-E cell dish, incubate at 37°C for 8h, aspirate the medium, and gently refill pre-warmed fresh DMEM complete medium containing 10% serum along the walls of the petri dish.
Day 4: Viral supernatants were collected 48h after transfection and filtered through a 0.22µm filter. The filtered supernatant was transferred to an ultracentrifuge tube and flattened, and centrifuged in an ultracentrifuge for 90 min at 4°C at 35,000 rpm. At the end of centrifugation, the supernatant was aspirated and discarded, and the precipitate obtained from each 30 mL of viral stock solution was resuspended with 300 µL of DMEM medium, and the resultant well-condensed mILS-ECAR retroviral solution was used for infection within 24 h or stored at -80°C.

### Example 10: Purification and culture of murine T cells

The biolegend brand of anti-mouse CD3 factor and anti-mouse CD28 factor were diluted to 1 µg/mL and 2 µg/mL, respectively, with PBS and added to 24-well plates at 500 µL per well, incubated in a 37°C incubator for 2h, and set aside. One Balb/c strain mouse was sacrificed by cervical disarticulation, and the spleen was removed and ground in an ultra-clean bench, lysed red with erythrocyte lysate (BD Biosciences), and filtered through a 0.45 µm filter to obtain a spleen single-cell suspension. T lymphocytes were isolated and purified using a Biolegend brand mouse CD3 T lymphocyte negative selection kit. T lymphocytes were isolated and purified using a solution containing 10% fetal bovine serum (Gibco), 1% penicillin-streptomycin solution (Beyotime Biotechnology), 1% 1M HEPES (Beijing Solarbio Science & Technology Co., Ltd), 1% MEN NEAA (Gibco), 1% 100 mM sodium pyruvate (Geno Biomedical Technology Co., Ltd.), 1‰ β-mercaptoethanol (Sigma-Aldrich) in 1640 complete medium was adjusted to a cell density of 1×10⁶/mL. 24-well plates incubated in a 37°C incubator were taken out, the liquid was pipetted off, and 1×10⁶ T-lymphocytes were added to each well, and the plate was incubated for 48 hours in a 37°C incubator.

### Example 11: mIL5-ECAR retrovirus infection of murine T cells with flow-through assay for virus infection efficiency

Using the mILS-ECAR concentrated virus obtained in Example 9, murine T cells were infected by centrifugal infection method to prepare CART cells, and the expression of mIL5-ECAR was detected by flow cytometry as follows:
After two days of T-cell activation culture, cell counting was performed and T-cell density was adjusted to 1 ×10⁶/well, 500 µL of fresh 1640 complete medium was added to each well and inoculated into a new 24-well plate.

Preparation of virus solution: 100 µL of virus concentrate from Example 9, 6 µg/mL Polybrene, and 400 µL 1640 complete medium were added to each well.

Centrifugation of infected T cells: place the 24-well plate in a centrifuge at 32°C, 1500g, and centrifuge for 2h.

After centrifugation, the infected T cells were centrifuged at 1600rpm for 5min, the supernatant was aspirated and discarded, and each well was seeded with 1mL of fresh 1640 complete medium in a new 24-well plate. The cells were incubated at 37 °C in an incubator with 5% CO2.

After 72h of culture, 5 ×10⁵ mIL5-ECAR T-cells and T-cells (control) were collected in flow tubes, respectively, washed once with PBS and the supernatant was discarded, and stained by adding a flow antibody to mIL5 from Biolegend for 30min avoiding light, followed by PBS washing, resuspension in 200 µL PBS, and detection by flow cytometer. The results are shown in FIG. 7. After 72h of reverse transcription infection of T cells with mIL5-ECAR obtained in Example 9, the expression efficiency of mIL5-ECAR is 56%.

### Example 12: Detection of the killing effect of mIL5-ECAR T on eosinophils after mIL5-ECAR T retroinfection in a mouse asthma model

Male Balb/c mice of 6-8 weeks SPF grade were taken and kept in the Animal Center of Zhejiang University. mIL5-ECAR T cells were injected into the tail vein at a dosage of 3 ×10⁶ per mouse, and resuspended in saline (Source Leaf Biologicals) at a volume of 200 µL per mouse, and the control group was injected with the same volume of saline.

Preparation of sensitization solution (ready-to-use): weigh 20 mg of ovalbumin (OVA, Sigma-Aldrich), dissolve it in 1 mL of saline, and call it solution A; take 0.4 mL of solution A into a 15 mL sterile centrifuge tube, add 9.6 mL of sterile saline and mix well, and call it solution B; take 2 mL of solution B into a new 15 mL centrifuge tube, add 2mL of Imject^{™} Alum Adjuvant (Thermo Scientific^{™}) and mix thoroughly to make the sensitization solution and set aside.

Sensitization: one week after mIL5-ECAR T cells were infused back, each mouse was injected intraperitoneally with 200 µL of sensitization solution, which was labeled as day 1; a second sensitization was performed on day 14 with the same solvent and volume.

Nebulization: followed by daily OVA nebulization on days 27, 28, and 29, respectively (volume of nebulizer was 10 mL, containing 130 mg OVA in saline as solvent).

Mouse treatment: mice were treated on day 30. Each mouse was injected intraperitoneally with 200 µL of 1.5% sodium pentobarbital sodium (Shandong Xiaya Chemical Industry Co., Ltd.). After the mice were anesthetized, the chest cavity was cut open and blood was taken from the heart into 1.5 mL EDTA anticoagulant tubes and placed on ice; the airway was exposed along with the lobes of the lungs on both sides, and the right lung was ligated with a fine thread near the hilum of the right lung with a small cut at the distal end of the airway, and the syringe was prepared (20 mL) manually sharpened blunt syringe needle and the barrel of a 1mL syringe). The syringe was inserted into the airway through the small hole, the needle was left in the airway, the syringe was withdrawn and clean PBS 400 µL was aspirated, after which it was slowly pushed from the airway into the lungs, and the push and pull was repeated for 2-3 times, and finally the aspirated fluid was pumped into a 1.5 mL EP tube and placed on ice, and the total fluid was bronchoalveolar lavage fluid (BALF). The above steps, repeated three times, yielded a total of approximately 1 mL of BALF. 400 µL of 4% formaldehyde solution (Sinopharm) was finally withdrawn and instilled into the left lung, and the airway was ligated to prevent formaldehyde from leaking out. The right quadruple lobe lungs were cut out and put into tissue homogenization tubes respectively, and put into liquid nitrogen for storage; the line of ligated airway was lifted, and the whole lobe lungs of the left side were cut out downward, and put into a 15mL centrifuge tube containing 13mL of 4% formaldehyde together with the heart.

After mixing the BALF solution, 20 µL was taken in a PCR tube, 20 µL of split red solution was added, mixed well, waited for 1 min, and counted under the microscope; the remaining BALF solution was centrifuged at 6000 rpm, 4°C, for 15 min, and the supernatant was collected and stored at -80°C; the cell precipitate was diluted with 200 µL of pre-cooled PBS (300 µL was added to the OVA group); and 100 µL of cell suspension was taken in a flow-through tube with 1 mL of PBS (including 1 tube of blank group, 6 tubes of single-label tubes), 400g, 4 °C , centrifuged for 5min, and the other 100 µL waited for dumping; after centrifugation, the supernatant was discarded, and 100 µL of pre-cooled PBS was added (containing 0.5 µL of flow antibodies, including CD45(PE-CY7, biolegend), SiglecF(PE. biolegend), F4/80 (APC-CY7, biolegend), CDllb (FITC, biolegend), CDllc (APC, biolegend), and DAPI (PB450), blown up well, protected from light, and incubated in the refrigerator at 4°C for 30 min; 1 mL of pre-cooled PBS was added to each tube at 400g, 4°C, centrifuged for 5 min. After the supernatant was discarded, 200 µL of pre-cooled PBS was added to each tube, and eosinophil expression was detected on the machine.

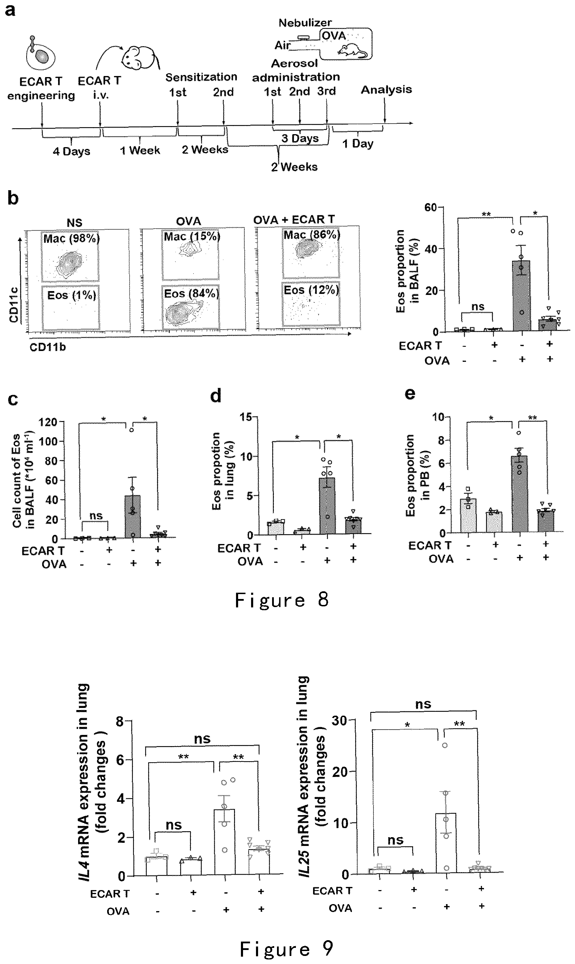

As shown in Figure 8, after treatment of Balb/c mice in the OVA asthma model (as shown in the modeling flowchart in Figure 8a) with mIL5-ECAR T, the proportion of eosinophils in the BALF fluid (as shown in the fourth group from the left to the right in the right panel of Figure 8b) as well as the absolute value of eosinophils in the BALF fluid (as shown in the fourth group from the left to the right in the right panel of Figure 8c) decreased significantly compared with that of the saline-injected OVA asthma model group (as shown in the right panel of Figure 8b and the third group from left to right in the bar graph of Figure 8c). Meanwhile, in the mILS-ECAR T-treated OVA asthma model group, the eosinophil ratio not only decreased in the BALF fluid, but also decreased in the lung tissues (as shown in the third and fourth groups from left to right in the bar graphs of Fig. 8d) and in the peripheral blood (as shown in the third and fourth groups from left to right in the bar graphs of Fig. 8e) also shows the same decrease change compared with that in the saline-injected OVA asthma model group, suggesting that mIL5-ECAR T can effectively reduce eosinophils in the OVA-induced asthma model.

### Example 13: Detection of the mitigating effect of mIL5-ECAR T on inflammatory factors after back-infusion into a mouse asthma model

Extraction of lung tissue RNA: One lobe of treated mouse lung tissue was selected, and 1 mL of Trizol (Takara) was added to the grinding beads. In a grinder at 60Hz speed, grind for 1min and repeat once. Let stand at room temperature for 5 min, blow and collect the lysate into a 1.5mL EP tube; add 0.2mL trichloromethane (Sinopharm) to each tube, mix well with shaking, and let stand at room temperature for 5 min; centrifuge for 15 min at 4°C, 12,000g; centrifuge and divide into 3 layers, the RNA was distributed into the upper aqueous phase, and 400 µL of the upper layer of the liquid was pipetted into new EP tubes; 0.5mL of isopropyl alcohol (Sinopharm) was added to each tube of RNA liquid, and the liquid was inverted up and down to mix gently, and left to stand at room temperature for 5min; 4 °C , 12000g, centrifugation for 10min; the supernatant was discarded, and the precipitate was left behind; 1mL of pre-cooled 75% ethanol was added to each tube, and the liquid was inverted up and down gently to make the precipitate float up, and the liquid should not be too hard to cause precipitate to be fragmented; 4°C, 9000g, centrifugation for 5min; the residue was removed with absorbent paper and dried for 5min at room temperature; Add appropriate amount of DEPC water and blow gently until the RNA is dissolved; use Nanodrop to determine the concentration and quality of RNA.

Reverse transcription of RNA: Reverse transcription kit from Takara was used, and the reaction system was as follows to get cDNA.

| | |
|---|---|
| Reverse transcription reaction system: 5×PrimeScript Buffer | 4µL |
| PrimeScript RT Enzyme Mix | 1µL |
| Random 6 mers(100uM) | 1µL |
| Oligo dT Primer(50uM) | 1µL |
| Total mRNA | 1000ng |
| DEPC water | up to 20µL |
| Reaction conditions: 37°C 15min | |
| 85°C 5sec | |

The mRNA levels of IL4, IL25 inflammatory factors were quantified by fluorescence quantitative PCR (RT-PCR) using Takara's fluorescence quantitative PCR kit with the following reaction system.

| | |
|---|---|
| RT-PCR reaction system: Taq Mix | 10µL |
| Primer F | 1µL |
| Primer R | 1µL |
| ddH20 | 6µL |
| cDNA | 2µL |

The reaction program is as follows:

| | | | | | |
|---|---|---|---|---|---|
| 94°C | 2 min | | | | |
| 94°C | 15 sec, | 60°C | 30 sec, | 72°C | 30 sec, for 40 cycles |

| | |
|---|---|
| 72°C | 10 min |
| 4°C | forever |

After the data were processed and analyzed, the results are shown in Figure 9. After treating Balb/c mice in the OVA model with mIL5-ECAR T (the fourth group from left to right in the bar graph of Figure 9), the mRNA levels of inflammation-related factors (IL4,IL25) in their lung tissues are significantly lower than those in the OVA model group injected with saline alone (e.g., the third group from the left to the right in the bar chart of Figure 9) and there is no significant difference from the untreated group (as shown in the first group from left to right in the bar graph of Fig. 9) and the group injected with mIL5-ECAR T alone (as shown in the second group from left to right in the bar graph of Fig. 9), and the results show a significant relief of airway inflammation.

### Example 14: Detection of relief of inflammation after mIL5-ECAR T back-infusion in a mouse asthma model

The severity of airway inflammation was determined by observing cellular infiltration of inflammatory cells around the airways of mouse lung tissues through lung histopathology.

Google Biotechnology Ltd. was commissioned to make lung tissue sections and Hematoxylin-Eosin (HE) staining was performed to facilitate the observation of inflammatory cell infiltration.

The specific semi-quantitative airway inflammation score was as follows:
Score 0: no inflammatory cell infiltration around the airway;
Score 1: occasional inflammatory cell infiltration around the airways;
Score 2: a thin layer of inflammatory cell infiltration around most of the airways or a localized thick layer of inflammatory cell infiltration;
Score 3: thick layer of inflammatory cell infiltration around most of the airways.

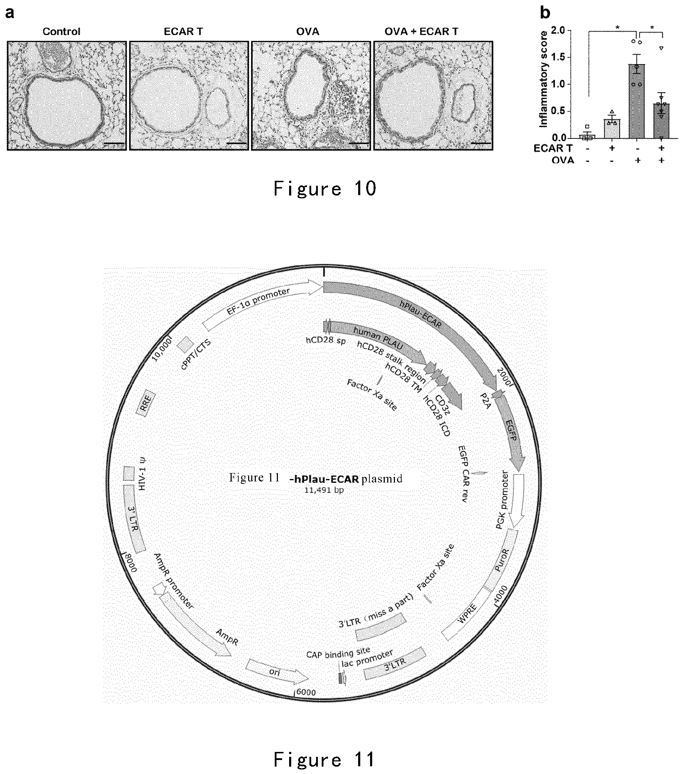

The results are shown in Figure 10, which clearly shows that the inflammatory cell infiltration in the representative images of HE sections of lung tissues of Balb/c mice from the mILS-ECAR T-treated OVA model (e.g., the fourth image from left to right in Figure 10a) is significantly less than that in the OVA model group injected with saline alone (e.g., the third image from left to right in Figure 10a). Further semiquantitative analysis of the data suggested that the inflammation level in the mIL5-ECAR T-treated OVA model group (as shown in the fourth panel from left to right in the bar graph of FIG. 10b) is more significantly alleviated compared to the saline-only injected OVA model group (as shown in the third panel from left to right in the bar graph of FIG. 10b).

### Example 15: Construction of hPlau-ECAR T primary mouse cells

The human Plau gene sequence was retrieved from the NCBI database.

A fragment of the coding region of the human Plau gene was synthesized by commissioning Tsingke Biotech Co., Ltd. According to the sequences of the coding region of the mouse CD8α signal peptide gene, the coding region of the human Plau gene, the coding region of the mouse CD28 hinge region gene, the coding region of the mouse CD28 transmembrane region gene, the coding region of the mouse CD28 intracellular region gene, and the coding region of the mouse CD3ζ intracellular region gene, the hPlau-ECAR gene sequences were ligated to the PMX retroviral plasmid by PCR and the Non-Ligase-Dependent Single Fragment Rapid Cloning Kit from Vazyme Biotech Co.,Ltd. The ligated product was transformed into the competent (DH5α), the plate was coated overnight, the single clone was picked and sent to Tsingke Biotech Co., Ltd. for sequencing, and the results of the sequencing results were compared to confirm whether the plasmid was constructed successfully or not.

The purified hPlau-ECAR lentiviral plasmid was extracted using the Plasmid Macro Extraction Kit from Cowin Biotech Co., Ltd. (an example of the plasmid map is shown in Figure 11).

Packaging and concentration of hPlau-ECAR retrovirus was performed according to Example 9.

Acquisition, activation and culture of murine T-lymphocytes was performed according to Example 10.

Construction of hPlau-ECAR T cells was performed according to Example 11.

### Example 16: Killing assay (luciferase assay) after co-culture of hPlau-ECAR T with target cells (simulated senescent cells) expressing human Plau receptor (hPlauR)

The hPlauR-293T target cells with luciferase were constructed by lentiviral infection method, and the hPlau-ECAR T cell killing ability was detected by luciferase luminescence intensity as follows:
Prepare WHB brand all-white 96-well plates, resuspend hPlauR-293T-Luciferase target cells, 293T-Luciferase cells with 100 µL of 1640 complete medium and adjust the cell density to 1×10⁴/well.

Resuspend hPlau-ECAR T cells prepared in Example 15 with 1640 Complete Medium medium, adjust the cell density to 2×10⁶/mL, gradient dilution and seed the plate with cell densities of 1×10⁵/well, 5×10⁴/well, and 2.5×10⁴/well, 100 µL per well.

In the experimental group, 100 µL of hPlauR-293T-Luciferase target cells were added to each well of 96-well plates, and 100 µL of hPlau-ECAR T cells with different cell densities; 100 µL of 293T-Luciferase target cells were added to each well of 96-well plates in the negative control group and 100 µL of hPlau-ECAR T cells with different cell densities; In the blank control group and positive control group, 100µL of hPlauR-293T-Luciferase and 100µL of 1640 complete medium were added to each well of the 96-well plates, which were mixed thoroughly and incubated in an incubator at 37°C with 5% CO2 for 12h.

The 96-well plates were removed after 12 h. In the positive control group, 1 µL of NP40 (Beijing Solarbio Science & Technology Co., Ltd) was added to each well and mixed thoroughly. 5 min later, the medium in the well plates was poured away, and 1 µL of coenzyme A, 1 µL of D-luciferase and 100 µL of PBS were added to each well, which was protected from light for 10 min, and the chemiluminescence intensity was measured using an M5 enzyme marker.

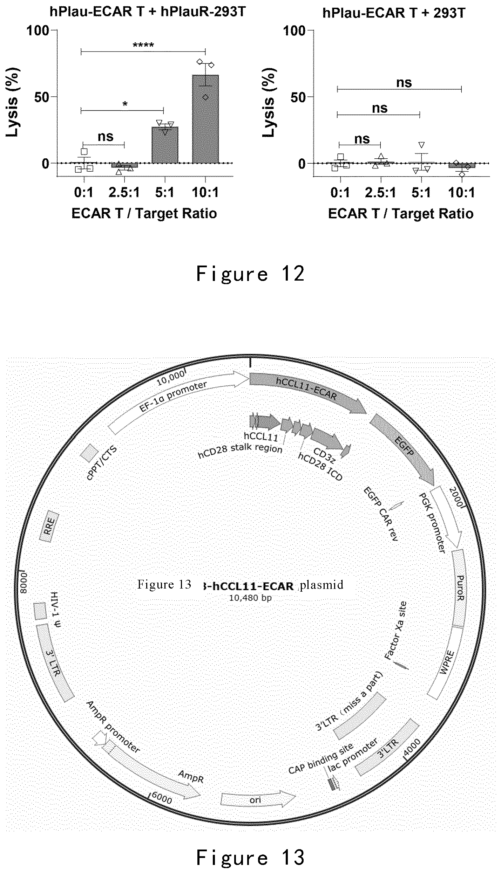

The results are shown in Figure 12. hPlau-ECAR T cells co-cultured with hPlauR-293T-Luciferase target cells showed dose-dependent enhancement of their killing ability as the effector-to-target ratio increased.

### Example 17: Construction of hCCL11-ECAR T primary mouse cells

The human CCL11 gene sequence was retrieved from the NCBI database.

A fragment of the coding region of the human-derived CCL11 gene was synthesized by commissioning Tsingke Biotech Co., Ltd. According to the sequences of the sequence of the coding region of the mouse CD8α signal peptide gene, the sequence of the coding region of the human CCL11 gene, the sequence of the coding region of the mouse CD28 hinge region gene, the sequence of the coding region of the mouse CD28 transmembrane region gene, the sequence of the coding region of the mouse CD28 intracellular domain gene, and the sequence of the coding region of the gene of the mouse CD3ζ intracellular region, the hCCL11-ECAR gene sequences were ligated to the PMX retroviral plasmid by PCR and the Non-Ligase-Dependent Single Fragment Rapid Cloning Kit from Vazyme Biotech Co.,Ltd. The ligated product was transformed into the competent (DH5α), the plate was coated overnight, the single clone was picked and sent to Tsingke Biotech Co., Ltd. for sequencing, and the results of the sequencing results were compared to confirm whether the plasmid was constructed successfully or not.

The purified hCCL11-ECAR lentiviral plasmid was extracted using the Plasmid Macro Extraction Kit from Cowin Biotech Co., Ltd. (an example of the plasmid map is shown in Figure 13).

Packaging and concentration of hPlau-ECAR retrovirus was performed according to Example 9.

Acquisition, activation and culture of murine T-lymphocytes was performed according to Example 10.

Construction of hCCL11-ECAR cells was performed according to Example 11.

### Example 18: Killing assay (luciferase assay) after co-culture of hCCL11-ECAR T with human-derived CCR3 receptor-expressing target cells (mimicking inflammatory cells (eosinophils))

The hCCR3-U2OS target cells with luciferase were constructed by lentiviral infection method, and the HCCl11-ECAR T cell killing ability was detected by luciferase luminescence intensity as follows:
Prepare WHB all-white 96-well plates, resuspend hCCR3-U2OS-Luciferase target cells with 100 µL of 1640 complete medium and adjust the cell density to 1×10⁴/well.

Resuspend hCCL11-ECAR T cells prepared in Example 17 with 1640 complete medium medium, adjust the cell density to 2×10⁶/mL, gradient dilution and seed the plate with cell densities of 2×10⁵/well, 1×10⁵/well, 5×10⁴/well, and 2.5×10⁴/well, 100 µL per well.

In the experimental group, 100 µL of hCCR3-U2OS-Luciferase target cells and 100 µL of different cell densities of hCCL11-ECAR T cells were added to each well of 96-well plates; in the blank control group and positive control group, 100 µL of hCCR3-U2OS-Luciferase target cells and 100 µL of 1640 complete medium were added to each well of 96-well plates, mixed thoroughly, and incubated in an incubator at 37°C with 5% CO2 for 12 hours.

The 96-well plate was removed after 12 hours. In the positive control group, 1 µL of NP40 was added to each well and mixed thoroughly. 5 min later, the medium in the well plate was poured away, and 1 µL of coenzyme A and 1 µL of D-luciferin and 100 µL of PBS were added to each well, and the chemiluminescence intensity was detected by using M5 enzyme marker after 10 min of protection from light.

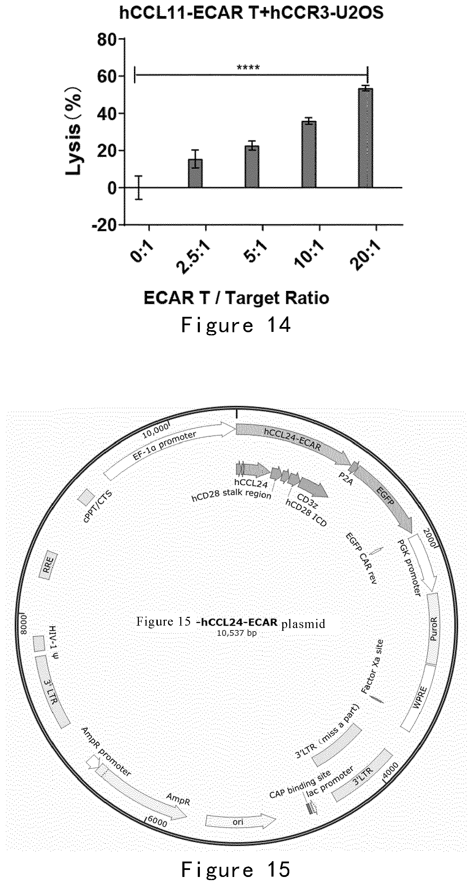

The results are shown in Figure 14. hCCL11-ECAR T cells co-cultured with hCCR3-U2OS-Luciferase target cells showed dose-dependent enhancement of their killing ability as the effector -to-target ratio increased.

### Example 19: Construction of hCCL24-ECAR T primary mouse cells

The human-derived CCL24 gene sequence was retrieved from the NCBI database.

A fragment of the coding region of the human-derived CCL24 gene was synthesized by commissioning Tsingke Biotech Co., Ltd. According to mouse CD8α signal peptide gene coding region sequence, human CCL24 gene coding region sequence, mouse CD28 hinge region gene coding region sequence, mouse CD28 transmembrane region gene coding region sequence, mouse CD28 intracellular domain gene coding region sequence, and gene coding region sequence of mouse CD3ζ intracellular region, thehCCL24-ECAR gene sequences were ligated to the PMX retroviral plasmid by PCR and the Non-Ligase-Dependent Single Fragment Rapid Cloning Kit from Vazyme Biotech Co.,Ltd. The ligated product was transformed into the competent (DH5α), the plate was coated overnight, the single clone was picked and sent to Tsingke Biotech Co., Ltd. for sequencing, and the results of the sequencing results were compared to confirm whether the plasmid was constructed successfully or not.

The purifiedhCCL24-ECAR lentiviral plasmid was extracted using the Plasmid Macro Extraction Kit from Cowin Biotech Co., Ltd. (an example of the plasmid map is shown in Figure 15).

Packaging and concentration of hCCL24-ECAR retrovirus was performed according to Example 9.

Acquisition, activation and culture of murine T-lymphocytes was performed according to Example 10.

Construction of hCCL24-ECAR cells was performed according to Example 11.

### Example 20: hCCL24-ECAR T killing assay after co-culture with human-derived CCR3 receptor-expressing target cells (luciferase assay)

The hCCR3-U2OS target cells with luciferase were constructed by lentiviral infection method and the hCCl24-ECAR T cell killing ability was detected by luciferase luminescence intensity as follows:
Prepare WHB all-white 96-well plates, resuspend hCCR3-U2OS-Luciferase target cells with 1640 complete medium, and adjust the cell density to 1×10⁴/well.

The hCCL24-ECAR T cells prepared in Example 17 were resuspended with 50 µL of 1640 complete medium medium, adjusted to a cell density of 2 × 10⁶/mL, gradient diluted and seeded with cell densities of 2 × 10⁵/well, 1 × 10⁵/well, 5 × 10⁴/well, and 2.5 × 10⁴/well in plates with 100 µL per well.

In the experimental group, 100 µL of hCCR3-U2OS-Luciferase target cells and 100 µL of different cell densities of hCCL24-ECAR T cells were added to each well of 96-well plates; in the blank control group and positive control group, 100 µL of hCCR3-U2OS-Luciferase target cells and 100 µL of 1640 complete medium were added to each well of 96-well plates, mixed thoroughly, and incubated in an incubator at 37°C with 5% CO2 for 12 hours.

The 96-well plate was removed at 12 hours. For the positive control group, 1 µL of NP40 was added to each well and mixed thoroughly. after 5 min, the medium in the well plate was poured away, and 1 µL of coenzyme A and 1 µL of D-luciferin and 100 µL of PBS were added to each well, and which was protected from light for 10 min, and the chemiluminescence intensity was measured using an M5 enzyme marker.

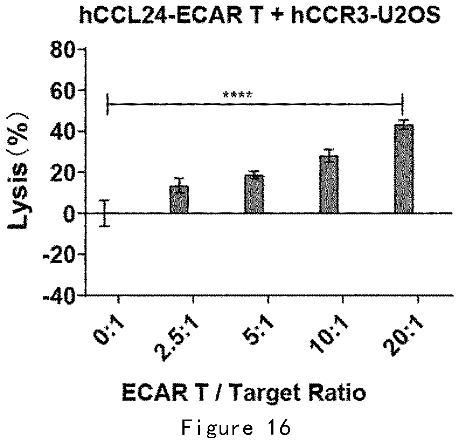

The results are shown in Figure 16. hCCL24-ECAR T cells co-cultured with hCCR3-U2OS-Luciferase target cells showed dose-dependent enhancement of killing ability with the increase of effector-target ratio.

All documents referred to in this invention are cited as references in this application as if each document were cited individually as a reference. It is further to be understood that after reading the foregoing teachings of the present invention, a person skilled in the art may make various alterations or modifications to the present invention, and these equivalent forms will likewise fall within the scope of the claims appended to this application.

## Claims

1. An endogenous chimeric antigen receptor CAR (ECAR), comprising an antigen-binding domain, which is an endogenous protein molecule.

2. The ECAR of claim 1, wherein the endogenous protein molecule is selected from the group consisting of: interleukin family, chemokine family, colony stimulating factor, growth factor, tumor necrosis factor superfamily, interferon family, tumor marker, senescent cells-associated factor, and a combination thereof.

3. The ECAR of claim 1, wherein the endogenous chimeric antigen receptor CAR (ECAR) has the structure shown in Formula I:
L-Z1-Z2-TM-C-CD3ζ (I)
wherein,
Each "-" is independently a linker peptide or peptide bond;
L is an optional signal peptide sequence;
Z1 is an antigen-binding domain, which is an endogenous protein molecule; and
Z2 is an absent or a hinge region;
TM is a transmembrane structural domain;
C is a co-stimulatory signaling molecule;
CD3ζ is a cytoplasmic signaling sequence derived from CD3ζ.

4. A nucleic acid molecule encoding an endogenous chimeric antigen receptor CAR (ECAR) of claim 1.

5. A vector, comprising a nucleic acid molecule of claim 4.

6. A host cell, comprising a vector of claim 5 or a chromosome incorporating an exogenous nucleic acid molecule of claim 4 or expressing an ECAR of claim 1.

7. A method of preparing an engineered immune cell, wherein the engineered immune cell expressing an ECAR of claim 1, comprising the step of transducing a nucleic acid molecule of claim 4 or a vector of claim 5 into a macrophage, a T-cell, or an NK-cell, thereby obtaining the engineered immune cell.

8. A pharmaceutical composition, comprising an ECAR of claim 1, a nucleic acid molecule of claim 4, a vector of claim 5, or a host cell of claim 6, as well as a pharmaceutically acceptable carrier, diluent or excipient.

9. Use of an ECAR of claim 1, a nucleic acid molecule of claim 4, a vector of claim 5, a host cell of claim 6, or a pharmaceutical composition of claim 8 for the preparation of a drug or formulation for (a) selective killing of a cell; and/or (b) treating a disease .

10. A kit for selective cell killing, comprising a container, and an ECAR of claim 1, a nucleic acid molecule of claim 4, a vector of claim 5, a host cell of claim 6, or a pharmaceutical composition of claim 8 in the container.
